(19) **Europäisches Patentamt**
**European Patent Office**
**Office européen des brevets**

(11) **EP 1 987 357 B1**

(12) **FASCICULE DE BREVET EUROPEEN**

(45) Date de publication et mention
de la délivrance du brevet:
**13.10.2010 Bulletin 2010/41**

(51) Int Cl.:
***G01N 33/53*** *(2006.01)* ***C07K 14/435*** *(2006.01)*
***C12N 9/64*** *(2006.01)*

(21) Numéro de dépôt: **07730937.5**

(22) Date de dépôt: **07.02.2007**

(86) Numéro de dépôt international:
**PCT/FR2007/000221**

(87) Numéro de publication internationale:
**WO 2007/090960 (16.08.2007 Gazette 2007/33)**

(54) **POLYPEPTIDES RECONNUS PAR DES ANTICORPS ANTI-TRICHINELLA, ET LEURS APPLICATIONS**

VON ANTI-TRICHINELLA-ANTIKÖRPERN ERKANNTE POLYPEPTIDE UND IHRE VERWENDUNG

POLYPEPTIDES RECOGNIZED BY ANTI-TRICHINELLA ANTIBODIES, AND USES THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **07.02.2006 FR 0601058**

(43) Date de publication de la demande:
**05.11.2008 Bulletin 2008/45**

(73) Titulaires:
• **Institute National de la Recherche Agronomique 75007 Paris (FR)**
• **Agence Française de Securité Sanitaire des Aliments 94700 Maisons-Alfort Cedex (FR)**
• **Jilin University Prc-Changchun 130-062 (CN)**

(72) Inventeurs:
• **BOIREAU, Pascal**
**F-91070 Bondoufle (FR)**
• **LIU, Mingyuan**
**Changchun 130062 (CN)**
• **FU, Baoquan**
**Lanzhou 730046 (CN)**
• **LE RHUN, Danielle**
**F-94370 Sucy en Brie (FR)**
• **BAHUON, Céline**
**F-94700 Maisons-Alfort (FR)**
• **VALLEE, Isabelle**
**F-94100 Saint Maur des Fosses (FR)**
• **LE GUERHIER, Franck**
**F-91800 Brunoy (FR)**

• **HERNANDEZ BELLO, Romel**
**Mexico, D.F. 3630 (MX)**
• **WU, Xiuping**
**130062 Changchun (CN)**

(74) Mandataire: **Vialle-Presles, Marie José et al Cabinet ORES 36, rue de St Pétersbourg 75008 Paris (FR)**

(56) Documents cités:
**US-A- 5 422 263**

• **DATABASE UniProt [Online] 1 mars 2004 (2004-03-01), "Newborn larvae-specific serine protease SS2" XP002401142 extrait de EBI accession no. UNIPROT:Q9BJL7 Database accession no. Q9BJL7 cité dans la demande**
• **DATABASE UniProt [Online] 5 juillet 2004 (2004-07-05), "Newborn larvae-specific serine protease SS2-1" XP002401143 extrait de EBI accession no. UNIPROT:Q6RUJ3 Database accession no. Q6RUJ3 cité dans la demande**
• **NAGANO ISAO ET AL: "MOLECULAR CLONING AND CHARACTERIZATION OF A SERINE PROTEINASE GENE OF TRICHINELLA SPIRALIS" JOURNAL OF PARASITOLOGY, AMERICAN SOC. OF PARASITOLOGISTS, WINSTON-SALEM,, US, vol. 89, no. 1, février 2003 (2003-02), pages 92-98, XP008069172 ISSN: 0022-3395**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- NAGANO I ET AL: "MOLECULAR CLONING AND CHARACTERIZATION OF A 21 KDA PROTEIN SECRETED FROM TRICHINELLA PSEUDOSPIRALIS" JOURNAL OF HELMINTHOLOGY, LONDON SCHOOL OF HYGIENS AND TROPICAL MEDICINE,, GB, vol. 75, no. 3, septembre 2001 (2001-09), pages 273-278, XP008069143 ISSN: 0022-149X
- ROMARIS FERNANDA ET AL: "A putative serine protease among the excretory-secretory glycoproteins of L1 Trichinella spiralis." MOLECULAR AND BIOCHEMICAL PARASITOLOGY. JUL 2002, vol. 122, no. 2, juillet 2002 (2002-07), pages 149-160, XP002400684 ISSN: 0166-6851
- LUN H M ET AL: "Characterization and cloning of metallo-proteinase in the excretory/secretory products of the infective-stage larva of Trichinella spiralis." PARASITOLOGY RESEARCH. MAY 2003, vol. 90, no. 1, mai 2003 (2003-05), pages 27-37, XP002400687 ISSN: 0932-0113
- BOIREAU P ET AL: "Trichinella antigens and immunodominant epitopes" 2004, MULTIDISCIPLINARITY FOR PARASITES, VECTORS AND PARASITIC DISEASES, VOL 1 MEDIMOND PUBLISHING CO, VIA RUBBIANI 6/2, 40124 BOLOGNA, ITALY, PAGE(S) 181-188 , 9TH EUROPEAN MULTICOLLOQUIUM OF PARASITOLOGY; VALENCIA, SPAIN; JULY 18 23, 2004 , XP008069099 ISSN: 88-7587-115-9(S)

**Description**

La présente invention concerne l'utilisation de nouveaux antigènes identifiés chez le parasite *Trichinella* dans le cadre du diagnostic et de la prévention de la trichinellose.

La trichinellose est une zoonose associée à la consommation de viande infestée par le parasite *Trichinella* (MURRELL *et al.,* 2000).

Ce nématode de la classe des *Adenophorea* appartient à la famille des *Trichinellidae* laquelle comprend 8 espèces et 3 génotypes apparentés en 2 groupes phylogénétiquement distincts : d'une part les trichines encapsulées *(T. spiralis; T. nativa; T. britovi; T. murrelli; T. nelsoni)* qui infestent les mammifères, et d'autre part les trichines non-encapsulées (*T. pseudospiralis; T. papuae; T. zimbabwensis)* qui infestent les mammifères, les oiseaux et les reptiles (GASSER et *al.,* 2004). Toutes ces espèces peuvent infester l'homme.

Le cycle biologique du parasite est auto-hétéroxène : il se déroule entièrement chez un même hôte qui est successivement hôte définitif (porteur de parasites adultes) et hôte intermédiaire (porteur de larves infestantes) (BOIREAU et *al.,* 2002). Le passage des larves infestantes d'un hôte à un autre est nécessaire pour la réalisation d'un nouveau cycle. Ce passage se fait par ingestion de viande crue ou peu cuite contaminée par les larves. Au cours de la digestion, celles-ci sont libérées, et pénètrent dans l'épithélium intestinal où elles vont muer en vers adultes (Ad) sexués. Les femelles fécondées expulsent ensuite des larves L1 Nouveau-Nées (L1NN) qui gagnent les muscles striés via la circulation lymphatique et sanguine. Ces larves L1NN pénètrent dans les cellules musculaires (stade de développement infestant L1M : Larve L1 Musculaire) dont elles provoquent la dédifférenciation en cellules nourricières entourées d'une capsule de collagène protectrice épaisse dans le cas des trichines encapsulées, très fine dans le cas des trichines dites non-encapsulées.

Alors que la trichinellose est asymptomatique chez l'animal, l'infestation humaine se traduit durant la phase intestinale initiale par des diarrhées associées à des nausées, vomissements et de violentes douleurs abdominales, alors que les symptômes liés à la phase d'invasion musculaire se caractérisent par l'association fièvre, oedème de la face et myalgies (CAPO & DESPOMMIER, 1996). Des atteintes oculaires, pulmonaires, gastro-intestinales, cardiaques et neurologiques peuvent venir compléter ce tableau clinique de la trichinellose dont l'évolution peut être mortelle. Le caractère chronique de l'infestation, marqué par la persistance de douleurs musculaires chez les patients, est associé à la survie du parasite au sein de la cellule nourricière.

Le traitement spécifique des trichinelloses humaines par des anthelminthiques est d'autant plus efficace que le diagnostic de l'infestation est posé précocement pour permettre une action contre tous les stades parasitaires et surtout avant la formation de la capsule protectrice de collagène autour des larves L1M (FOURESTIE *et al.*, 1988).

Les données épidémiologiques ont démontré une distribution géographique du parasite sous toutes les latitudes associée à un mode de transmission impliquant de nombreuses espèces de la faune sauvage qui entretiennent aussi un cycle d'infestation domestique principalement représenté par le porc (DUPOUY-CAMET, 2000).

Les épidémies de trichinellose humaine, zoonose émergente ou ré-émergente, constituent un véritable problème de santé publique à travers le monde en raison des habitudes alimentaires et des contrôles sanitaires pas toujours efficaces (MURRELL & POZIO, 2000). Ces épidémies impliquent essentiellement la viande de porc et de sanglier ainsi que celle de cheval (BOIREAU et *al.,* 2000).

La prévention de la contamination humaine passe donc par une cuisson à coeur de la viande et l'amélioration des conditions d'élevage et/ou de contrôle des trichinelloses animales (porc, cheval, sanglier et autres espèces animales sauvages sensibles à *Trichinella*) (BOIREAU *et al.,* 2002).

Les techniques de dépistage de la trichinellose se divisent en deux catégories : 1) la détection directe des larves L1M, par trichinoscopie (observation microscopique d'un fragment de viande), ou après digestion artificielle d'échantillons de muscles, et 2) la détection indirecte par différentes méthodes immunologiques, permettant de détecter des anticorps dirigés contre les antigènes de *Trichinella.*

A chacun des stades de développement du parasite : adulte (Ad), larve nouveau-née (L1NN), et larve musculaire (L1M), correspond un profil antigénique particulier.

Ce sont des préparations antigéniques issues de larves du stade L1M qui sont actuellement utilisées pour l'immunodiagnostic. En effet, les fractions antigéniques des deux stades précoces Ad et L1NN sont difficile à purifier, et des antigènes immunodominants associés à l'un et/ou l'autre de ces deux stades n'avaient pas pu être identifiés jusqu'à présent.

On utilise principalement soit des préparations d'antigène total soluble, obtenues par lyse des larves, centrifugation du lysat, et récupération du surnageant, soit plus fréquemment, des antigènes d'excrétion/sécrétion (antigènes E/S).

Les antigènes d'excrétion-sécrétion sont produits lors de la mise en survie des larves L1M dans un milieu de culture ; ils proviennent d'un organe particulier, dénommé le stichosome, qui est constitué d'une cinquantaine de cellules discoïdes, les stichocytes. Les stichocytes contiennent des granules dont le contenu est évacué par un canalicule dans la lumière de l'oesophage du parasite. Ce contenu qui est très fortement antigénique, constitue une partie des antigènes d'excrétion sécrétion. Ces antigènes forment un mélange complexe de protéines, contenant notamment un groupe de glycoprotéines (dénommées antigènes TSL1) portant une molécule glucidique particulière, connue uniquement chez *Trichinella* et présente chez toutes les espèces de ce parasite, le beta-tyvelose.

Les préparations d'antigènes d'excrétion-sécrétion qui sont actuellement utilisées comme référence en matière d'immunodiagnostic de la trichinellose sont obtenues à partir de milieu de culture de larves L1M de *Trichinella spiralis.* Après 18 à 20 heures de culture, le milieu est récupéré par filtration, puis concentré (GAMBLE *et al.,* 1983; GAMBLE *et al.,* 1988).

Les préparations d'antigène total soluble ont comme principal inconvénient leur manque de spécificité. Des réactions antigéniques croisées avec d'autres parasitoses sont fréquemment observées. Les antigènes d'excrétion-sécrétion permettent d'obtenir une meilleure spécificité. Cependant, dans les deux cas, il est délicat de produire en quantité importante des lots standardisés d'antigène.

Les séquences d'ADN encodant l'antigène E/S, et leurs utilisations pour produire des antigènes utiles pour le diagnostic ou la vaccination contre l'infection par *T.spiralis,* sont divulguées dans le brevet US5422263.

Les antigènes de *Trichinella* et leurs épitopes immunodominants sont passés en revue par Boireau et al., MULTIDISCIPLINARITY FOR PARASITES, VECTORS AND PARASITIC DISEASES, VOL 1 MEDIMOND PUBLISHING CO, VIA RUBBIANI 6/2, 40124 BOLOGNA, ITALY, pages 181-188; 2004.

La structure saccharidique contenant du beta-tyvelose, qui représente un épitope immunodominant des préparations d'antigène E/S (REASON *et al.,* 1994; Brevets US 5541075 et 5707817) a été synthétisée chimiquement, son utilisation pour l'immunodiagnostic de *Trichinella* a été proprosée.

Ce réactif présente une bonne spécificité, mais sa sensibilité apparaît plus faible que celle des préparations d'antigène E/S. En outre, la synthèse de cette structure par voie chimique demeure coûteuse et lourde à mettre en oeuvre.

Un autre problème rencontré dans le cadre du diagnostic sérologique de *Trichinella* est l'existence d'une « fenêtre aveugle » de détection correspondant aux stades précoces de l'infestation, qui se traduit par des résultats faussement négatifs. En outre, chez le cheval, une disparition progressive des anticorps a été observée 25 semaines après infestation.

Les Inventeurs ont entrepris d'identifier des antigènes immunodominants associés aux stades précoces de l'infestation par *Trichinella,* et utilisables pour le diagnostic sérologique de la trichinellose, afin de fournir des moyens d'obtenir une détection précoce, spécifique, et sensible des infestations à *Trichinella,* aussi bien chez l'homme que chez les animaux. Dans ce but, ils ont recherché s'il existait, parmi les produits des gènes exprimés par *Trichinella* au stade L1NN et/ou au stade Ad, des protéines qui posséderaient les propriétés antigéniques souhaitées.

Dans ce cadre, ils ont découvert qu'une protéine de *Trichinella spiralis,* qui fait partie des protéines exprimées spécifiquement au stade L1NN chez cet organisme, constituait un antigène immunodominant, permettant une détection précoce de la réponse humorale dirigée contre *Trichinella* et qu'elle était en outre conservée entre différentes espèces de *Trichinella.*

Cette protéine sera dénommée ci-après NBL1. La séquence complète d'ADNc codant pour cette protéine ainsi que la séquence polypeptidique qui en est déduite sont respectivement accessibles sur Genbank sous les numéros AF331160 et AAK16520 (alias Swissprot Q9BJL7); ces séquences sont annotées comme « sérine protéase SS2, spécifique des larves nouvelles nées ». Ces séquences sont également respectivement reproduites dans le listage de séquences en annexe sous les numéros SEQ ID NO: 1 et SEQ ID NO: 2. Une séquence partielle d'ADNc codant pour cette protéine ainsi que la séquence polypeptidique qui en est déduite sont respectivement accessibles sur Genbank sous les numéros AY491941 et AAR36900 (alias Swissprot Q6RUJ3).

Les Inventeurs ont en outre montré que l'immunoréactivité associée à la réponse humorale dirigée contre NBL1 était localisée sur la partie C-terminale de cette protéine, et identifié un épitope immunodominant responsable de cette réactivité.

D'autre part, les Inventeurs ont identifié, à partir d'une banque d'ADNc des stades précoces mélangés Ad+L1NN de *T. spiralis* un nouveau gène, dénommé ci-après 411.

La séquence de ce gène est représentée dans le listage de séquences en annexe sous le numéro SEQ ID NO: 3, et celle de son produit de traduction sous le numéro SEQ ID NO: 4. Ce produit de traduction de ce gène est apparenté (78,7% d'identité) à un antigène E/S, dénommé protéine Tp21-3, identifié chez *T. pseudospiralis* (AAF79206 ; NAGANO et *al.,* 2001), ainsi qu'au produit de traduction de l'ORF 17.20 hypothétique de *T. spiralis* (AAB48489), avec lequel il présente 86,6% d'identité.

Le produit de traduction du gène 411 permet également de détecter à un stade précoce, la réponse humorale dirigée contre différentes espèces de *Trichinella.*

En outre chacun des antigènes NBL1 et 411 a permis, lors des essais effectués, de détecter des animaux infestés par *Trichinella* qui n'étaient détectés ni par l'autre antigène, ni par l'antigène E/S, au moins pendant la période J15-J30 post-infestation (pi).

L'association de l'antigène NBL1 (ou d'un épitope immunodominant de celui-ci) avec l'antigène 411 permet donc d'améliorer la sensibilité du diagnostic, notamment aux stades précoces de l'infestation (15 à 20 jours après l'infestation).

La présente invention a en conséquence pour objet l'utilisation d'un polypeptide antigénique reconnu par des anticorps anti-*Trichinella* comme réactif pour la détection d'anticorps anti-*Trichinella* dans un prélèvement biologique, caractérisée en ce que ledit polypeptide est choisi parmi :

a) un polypeptide comprenant un épitope immunodominant de l'antigène NBL1, lequel épitope est défini par la séquence PSSGSRPTYP (SEQ ID NO: 5);

b) un polypeptide, également dénommé ci-après antigène 411, comprenant les acides aminés 25-175 de la séquence SEQ ID NO: 4 (qui représentent la forme mature de la protéine 411), ou comprenant une séquence présentant au moins 80%, et par ordre de préférence croissante, au moins 85%, 90%, ou 95% d'identité avec la séquence des acides aminés 25-175 de la séquence SEQ ID NO: 4.

La présente invention a plus particulièrement pour objet un procédé de détection de la présence d'anticorps anti-*Trichinella* dans un échantillon biologique, lequel procédé est caractérisé en ce qu'il comprend :

- la mise en contact dudit échantillon biologique avec un ou plusieurs polypeptide(s) a) et/ou un ou plusieurs polypeptide(s) b), tels que définis ci-dessus, dans des conditions permettant la formation d'un complexe antigène/anticorps avec les anticorps anti-*Trichinella* éventuellement présents dans ledit échantillon ;
- la détection, par tous moyens appropriés, du complexe antigène/anticorps éventuellement formé.

Généralement, ledit prélèvement biologique est un prélèvement de sérum. Il peut être obtenu à partir de tout sujet (mammifères, oiseau ou reptile) appartenant à une espèce pouvant être infestée par *Trichinella,* et chez lequel on souhaite détecter la présence de ce parasite. Avantageusement, il s'agit d'un prélèvement obtenu à partir d'un mammifère, par exemple d'un animal d' élevage, ou d'un patient humain.

Avantageusement, on utilise un mélange, comprenant un ou plusieurs polypeptide(s) a) et/ou un ou plusieurs polypeptide(s) b) tels que définis ci-dessus.

Cette association permet notamment d'élargir le spectre de réactivité, par rapport à chacun des polypeptides utilisé individuellement.

Les polypeptides a) définis ci-dessus, à l'exclusion de l'antigène NBL1 entier identifié par le numéro d'accès Genbank AAK16520, et de son fragment identifié par le numéro d'accès Genbank AAR36900, font également partie, en tant que tels, de l'objet de la présente invention.

Parmi ces polypeptides conformes à l'invention, on citera notamment les polypeptides contenant une ou plusieurs des séquences suivantes : la séquence : PSSGSRPTYPSSGSR (SEQ ID NO: 6); la séquence PSSGSRPTYPYTGSR (SEQ ID NO: 7); la séquence RPTSPSSGSRPTYPS (SEQ ID NO: 8).

Ceci englobe par exemple des fragments de la région C-terminale de l'antigène NBL1 : on citera notamment les fragments contenant la séquence suivante,

```
ENSPEGTVKWASKEDSPVDLSTASRPTNPYTGSRPTSPSSGSRPTYPSSGSRPTSPSSGSR
PTYPSSGSRPTYPSSGSRPTYPYTGSRPTPQKPVFPSYQKYPPAVQKYIDSLPSGTQGTLE
YTVTQNGVTTTT (SEQ ID NO:11)
```

qui correspond aux acides aminés 326-459 de la séquence SEQ ID NO: 2 ; et des sous-fragments de cette séquence SEQ ID NO: 11, notamment ceux comprenant la séquence suivante :

PSSGSRPTYPSSGSRPTSPSSGSRPTYPSSGSRPTYPSSGSRPTYP (SEQ ID NO: 9)

qui correspond aux acides aminés 363-409 de la séquence SEQ ID NO: 2, et, plus particulièrement, ceux comprenant la séquence suivante :

```
SRPTNPYTGSRPTSPSSGSRPTYPSSGSRPTSPSSGSRPTYPSSGSRPTYPSSGSRPTYPY
TGSRPT (SEQ ID NO: 10)
```

qui correspond aux acides aminés 349-415 de la séquence SEQ ID NO: 2.

Les polypeptides b) définis ci-dessus, à l'exception de ceux identifiés par les numéros d'accès GenBank AAF79206 et AAB48489 font partie, en tant que tels, de l'objet de la présente invention. Des polypeptides préférés sont notamment le polypeptide de séquence SEQ ID NO: 4, ou le polypeptide correspondant aux acides aminés 25-175 de la séquence SEQ ID NO: 4, ainsi que les polypeptides présentant au moins 90%, ou de préférence au moins 95% d'identité avec la séquence SEQ ID NO: 4, ou avec la séquence des acides aminés 25-175 de la séquence SEQ ID NO: 4.

La présente invention englobe notamment des polypeptides chimériques comprenant une ou plusieurs copies de la séquence PSSGSRPTYP (SEQ ID NO: 5), ou d'un fragment de l'antigène NBL1 contenant cette séquence et/ou une ou plusieurs copies d'un polypeptide b) tels que définis ci-dessus, éventuellement fusionnés à une ou plusieurs autres séquence(s) hétérologue(s).

La présente invention a également pour objet les polynucléotides codant pour les polypeptides conformes à l'invention, ainsi que des vecteurs recombinants comprenant lesdits polynucléotides, et des cellules-hôtes transformées par lesdits vecteurs.

La présente invention a également pour objet une composition comprenant un ou plusieurs polypeptide(s) a) et un ou plusieurs polypeptide(s) b), tels que définis ci-dessus, ainsi qu'une composition comprenant un ou plusieurs polynucléotide(s) codant pour le(s)dit(s) polypeptide(s).

Les polypeptides a) et b) définis ci-dessus peuvent être utilisés dans le cadre de différentes méthodes de détection d'anticorps, qui sont connues en elles-mêmes. A titre d'exemples, on citera notamment les méthodes de type ELISA (direct, indirect ou sandwich), les méthodes de microagglutination sur billes, ainsi que les méthodes de transfert électrophorétique couplé à un immunomarquage.

La présente invention a également pour objet un nécessaire pour la détection de la présence d'anticorps anti-*Trichinella* dans un échantillon biologique, caractérisé en ce qu'il comprend un ou plusieurs polypeptide(s) a) et/ou un ou plusieurs polypeptide(s) b) tels que définis ci-dessus, et, le cas échéant, des tampons et réactifs appropriés pour la constitution d'un milieu réactionnel permettant la formation d'un complexe antigène/anticorps, et, optionnellement, des moyens de détection dudit complexe antigène/anticorps.

Avantageusement, ledit nécessaire comprend un polypeptide a) et/ou un polypeptide b) tels que définis ci-dessus, immobilisé(s) sur un support solide. A titre d'exemples non-limitatifs de supports solides utilisables, on citera des plaques de microtitration, des billes, des microbilles ou microparticules, des bandelettes, etc....

Ledit nécessaire peut également comprendre des échantillons de référence, tels qu'un ou plusieurs sérum(s) négatif(s) et un ou plusieurs sérum(s) positif(s).

La présente invention a également pour objet l'utilisation d'un polypeptide a) ou d'un polypeptide b), tels que définis ci-dessus, pour la préparation d'anticorps spécifiquement dirigés contre ledit polypeptide.

Ces polypeptides peuvent être utilisés dans le cadre de différentes méthodes, connues en elles-mêmes, de préparation d'anticorps.. Ils peuvent par exemple (éventuellement après addition d'un adjuvant approprié) être utilisés pour l'immunisation d'un animal. Ils peuvent également être greffés sur un support de chromatographie d'affinité, afin de permettre de purifier, à partir d'un liquide biologique, les anticorps spécifiquement dirigés contre le polypeptide concerné. Le liquide biologique peut être par exemple le sérum d'un animal préalablement immunisé avec le polypeptide concerné, ou un surnageant d'hybridome ; il peut s'agir également du sérum d'un animal infesté par *Trichinella* à partir duquel on souhaite isoler une sous-population d'anticorps spécifiquement dirigés contre le polypeptide concerné.

La présente invention englobe également tout anticorps spécifiquement dirigé contre un polypeptide a) ou un polypeptide b) tels que définis ci dessus. Il peut s'agir d'anticorps polyclonaux ou monoclonaux. Des anticorps préférés sont ceux reconnaissant l'épitope PSSGSRPTYP (SEQ ID NO: 5).

Des anticorps spécifiquement dirigés contre un polypeptide peuvent être obtenus par diverses techniques connues en elles-mêmes, et notamment par les méthodes classiques comprenant l'immunisation d'un animal avec le polypeptide concerné (éventuellement additionné d'un adjuvant approprié), et la récupération de son sérum (pour la production d'anticorps polyclonaux), ou de ses cellules lymphocytaires(pour la production d'anticorps monoclonaux).

Les polypeptides a) et b) définis ci-dessus, ainsi que les polynucléotides codant pour ces polypeptides peuvent être utilisés pour la préparation de compositions immunogènes, et notamment de vaccins anti-Trichinella.

La présente invention a également pour objet une composition immunogène, comprenant un ou plusieurs polypeptide(s) a) et/ou un ou plusieurs polypeptide(s) b) tels que définis ci dessus, ou un ou plusieurs polynucléotide (s) codant pour le(s)dit(s) polypeptide(s), associé(s) à un ou plusieurs adjuvant(s) permettant d'améliorer la réponse immunitaire.

Selon un mode de réalisation préféré d'une composition immunogène conforme à l'invention, il s'agit d'un vaccin.

Une grande variété d'adjuvants permettant d'augmenter l'immunogénicité des peptides sont connus en eux mêmes de l'homme de l'art : on citera, à titre d'exemples d'adjuvants, l'alum (hydroxyde d'aluminium), l'adjuvant complet ou incomplet de Freund (AIF), les liposomes, ainsi que les virosomes (enveloppes virales reconstituées), les dérivés peptidiques de l'acide muramique, etc. Dans le cas d'un vaccin, on choisira bien entendu un adjuvant pharmacologiquement acceptable ; à titre d'exemples d'adjuvants préférés on citera des adjuvants de type émulsion « eau dans l'huile », par exemple les adjuvants commercialisés par la société SEPPIC sous les dénominations MONTANIDE ISA 70 et MONTANIDE ISA 775, et qui sont également décrits dans les Brevets EP 480 982, EP 825 875, US 5422109, US 6251407, US 6610309.

Le cas échéant, notamment dans le cas de peptides courts ($\leq$ 30 acides aminés), le(s)dit(s) polypeptide(s) peu (ven)t être couplé(s) à une protéine porteuse.

A titre d'exemples de protéines porteuses, on citera notamment la KLH (keyhole limpet hemocyanin), l'albumine du sérum bovin (BSA), l'ovalbumine, l'anatoxine tétanique ou diphtérique. On peut également former une composition multiépitopique, en associant plusieurs copies d'un même peptide entre elles, et éventuellement avec d'autres épitopes peptidiques, sous forme de polypeptides chimériques, ou par l'intermédiaire d'une chaîne polymérique, par exemple une polylysine.

Si l'on utilise comme immunogène un polynucléotide, la composition immunogène peut se présenter sous forme d'un vecteur recombinant dans lequel est (sont) inséré(s) le (les) polynucléotide(s) à administrer. On peut utiliser par exemple des vecteurs viraux tels que les poxvirus, les adénovirus, les rétrovirus, les lentivirus, les herpesvirus, et les AAV (adeno-associated virus), etc.... Elle peut également se présenter sous la forme d'une bactérie non-pathogène, transformée par un ou plusieurs vecteurs d'expression contenant le(s)dit(s) polynucléotide(s). On peut également administrer directement le (les) polynucléotide(s), sous forme d'ADN nu, ou l'incorporer dans des liposomes. Dans le cas d'un vaccin, on utilisera de préférence une bactérie non-pathogène (par exemple un lactobacille, ou une souche non pathogène *d'Escherichia coli* ou de *Salmonella suis*), ou un vecteur dérivé d'une souche virale vaccinale ; par exemple un vecteur dérivé d'une souche vaccinale du virus de la pseudorage (maladie d'Aujeszky).

La présente invention sera mieux comprise à l'aide du complément de description qui va suivre, qui se réfère à des exemples illustrant l'utilisation des antigènes NBL1 et 411, pour l'immunodiagnostic précoce de la trichinellose.

**Légende des figures**

**Figure 1** : Séquence de la protéine THX-NBL1(Cterm).
La séquence provenant de NBL1 est indiquée en caractères gras et les séquences provenant du plasmide pET102 sont indiquées en italique.
**Figure 2** : Immunoréactivité de la protéine THX-NBL1(Cterm). M: Marqueur de poids moléculaires ; 1 : sérum de porc négatif ; 2 : sérum de porc expérimentalement infesté par 20000 L1M de *T. spiralis.*
**Figure 3** : Comparaison des cinétiques d'apparition des anticorps anti-*Trichinella* détectés par ELISA THX-NBL1 (Cterm) ou par ELISA Ag E/S dans des sérums de porcs infestés par T. *spiralis.*
A : Détection par ELISA THX-NBL1 (Cterm) ; B : Détection par ELISA Ag E/S
En abscisse : nombre de jours après infestation ; en ordonnée: pourcentage de réactivité. Le seuil de détection des ELISA est marqué d'un trait noir (44% pour l'ELISA THX-NBL1(Cterm) et 14% pour l'ELISA Ag E/S). La moyenne et les écarts-types du rapport échantillon/contrôle positif sont indiqués pour chaque groupe de porcs infestés.

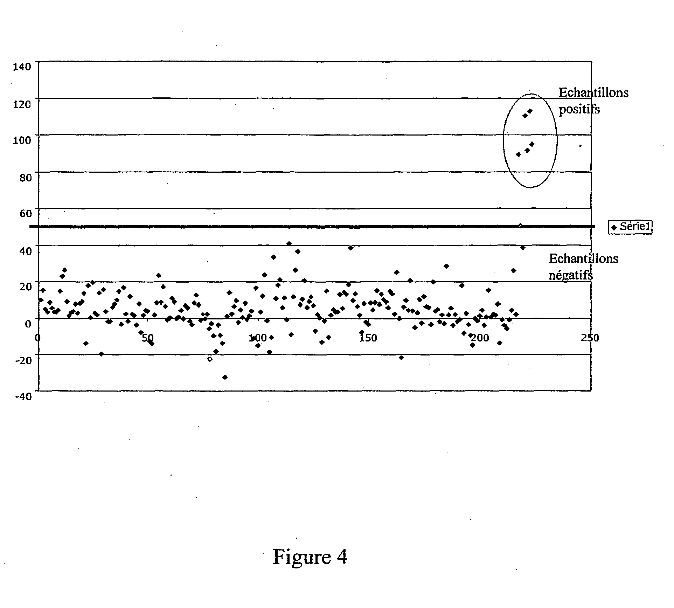

**Figure 4** : Spécificité de l'ELISA NBL1(Cterm).
En abscisse : cohorte de 230 échantillons négatifs provenant de porcs industriels ou de porcs plein air. Les 5 échantillons positifs proviennent de sérums de porcs expérimentalement infestés. Le seuil est égal à 2 fois la valeur moyenne des échantillons négatifs. En ordonnée : pourcentage de réactivité.

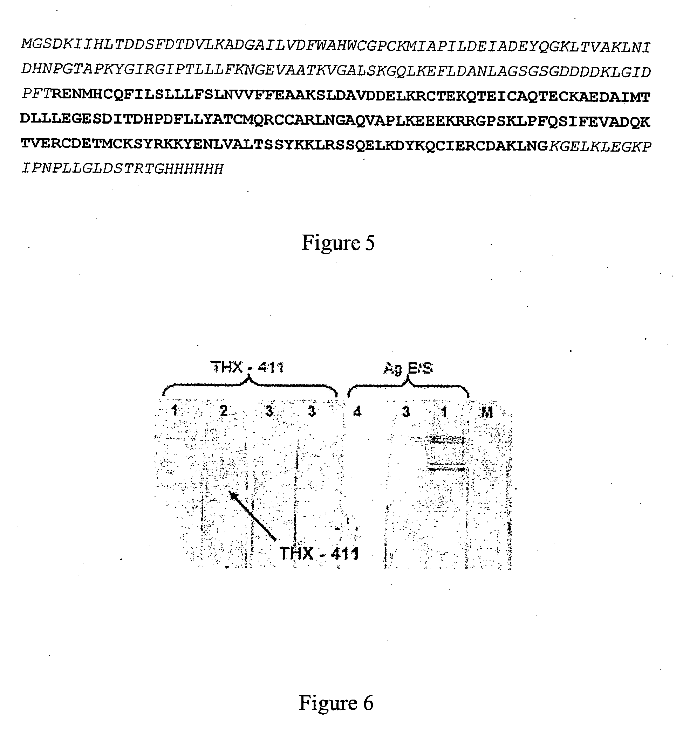

**Figure 5** : Séquence de la protéine THX-411.
La séquence provenant de 411 est indiquée en caractères gras. Les séquences provenant du plasmide sont indiquées en italique.
**Figure 6** : Immunoréactivité de la protéine THX-411.
1: sérum positif 50j pi; 2: sérum positif 30j pi; 3: sérum négatif -5j; 4: contrôle conjugué. M: Marqueur de poids moléculaires.

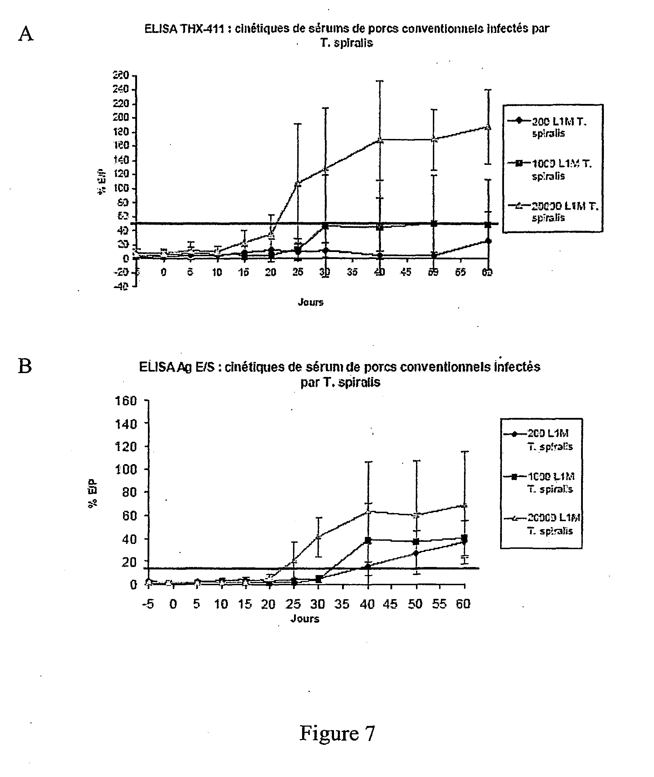

**Figure 7** : Comparaison des cinétiques d'apparition des anticorps anti-*Trichinella* détectés par ELISA THX-411 ou par ELISA Ag E/S dans des sérums de porcs infestés par T. *spiralis.*
A : Détection par ELISA THX-411 ; B : Détection par ELISA Ag E/S
En abscisse : nombre de jours après infestation ; en ordonnée: pourcentage de réactivité. Le seuil de détection des ELISA est marqué d'un trait noir (52% pour l'ELISA THX-411 et 14% pour l' ELISA Ag E/S). La moyenne et les écarts-types du rapport échantillon/contrôle positif sont indiqués pour chaque groupe de porcs infestés.

**EXEMPLE 1 : PRODUCTION DE LA PROTEINE RECOMBINANTE THX-NBL1(CTERM), CONTENANT UNE PORTION C-TERMINALE DE NBL1.**

Un immunocriblage d'une banque d'ADNc L1NN de *Trichinella spiralis* a été réalisé avec un sérum de porc obtenu 35 jours après infestation expérimentale avec 10000 L1M de *T. spiralis.* Le séquençage des clones reconnus par ce sérum a permis de déterminer que la majeure partie d'entre eux codaient pour une même protéine.

Cette protéine est une protéase à sérine putative, dont la séquence d'ADNc et la séquence en acides aminés déduite sont respectivement disponibles sur Genbank sous les numéros AF331160 et AAK16520, et sont également reproduites ici sous les numéros SEQ ID NO: 1 et SEQ ID NO: 2. Elle est dénommée ici NBL1.

Une portion de la partie C terminale de la protéine a été amplifiée à l'aide des oligonucléotides NBL1CtermF (5'-CACCGAAAATTCTCCTGAAGGA-3') (SEQ ID NO: 12) et NBL1CtermR (5'-TGTTGTTGTAGTAACTCC-3') (SEQ ID NO: 13), et de l'ADN polymérase AccuPrime Pfx DNA polymerase (Invitrogen), et clonée dans le plasmide pET102D/ topo en utilisant le kit d'expression « Champion pET102 Directional TOPO » selon les recommandations du fabricant (Invitrogen).

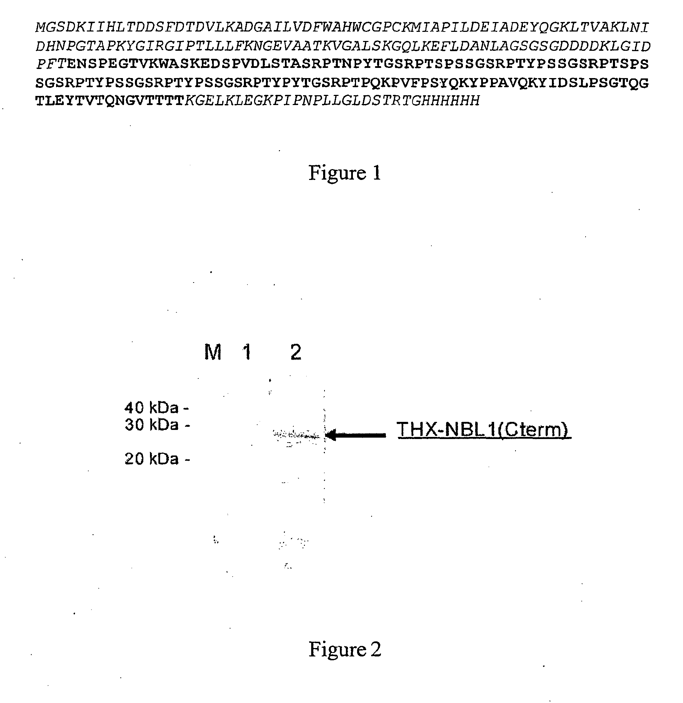

Le plasmide recombinant obtenu, dénommé pET102-NBL1(Cterm) code pour une protéine de fusion thioredoxine-NBL1(Cterm) (THX-NBL1(Cterm), de 291 AA, portant en position C-terminale une étiquette poly-histidine. La séquence de cette protéine de fusion est représentée sur la Figure 1.

La protéine de fusion THX-NBL1(Cterm) a été exprimée dans des bactéries *E. coli* BL21 Star (DE3), BL21 (DE3) pLys (Invitrogen) transformées par le plasmide pET102-NBL1(Cterm), et purifiée par chromatographie d'affinité en conditions dénaturantes sur colonne Ni-NTA (Ni-NTA spin columns kit ; Ni-NTA beads), en utilisant le protocole préconisé par le fournisseur (Qiagen).

La protéine de fusion THX-NBL1(Cterm) purifiée apparaît, après électrophorèse en conditions dénaturantes (SDS-PAGE) sous forme d'une bande à la taille attendue de 31,1 kDa.

L'immunoréactivité de la protéine THX-NBL1(Cterm) vis-à-vis d'un sérum de porc indemne de trichinellose et d'un sérum de porc infesté par 20000 larves L1M de T. *spiralis* prélevé 60 jours après l'infestation, été analysée par transfert de Western.

Après électrophorèse en conditions dénaturantes (SDS-PAGE), la protéine a été électro-transférée sur une membrane Hybond P (PVDF) suivant les instructions du fournisseur (Amersham). Les membranes ont été pré-hybridées pendant 1 h en TBS-T (20 mM Tris-HCl pH 7,5, 150 mM NaCl, 0,1% Tween 20) et 5% de lait écrémé. Après 2 lavages en TBS-T pendant 1 min, puis 3 lavages en TBS-T pendant 5 min, les membranes ont été incubées pendant 1 h avec le sérum de porc dilué au 1/200 en TBS. Après lavage, les membranes ont été incubées pendant 20 min avec l'anticorps secondaire de lapin anti-IgG de porc marqué à la phosphatase alcaline (A1192, Sigma) dilué au 1/30000, puis avec le substrat NBT/BCIP (E116, Interchim) pur pendant 30 min pour la révélation du marquage.

Les résultats sont illustrés par la Figure 2.

On observe une très forte immunoréactivité de THX-NBL1(Cterm) avec le sérum du porc infesté par T. *spiralis.*

## EXEMPLE 2 : UTILISATION DE THX-NBL1(CTERM) POUR DETECTER LA REPONSE HUMORALE DIRIGEE CONTRE *TRICHINELLA.*

La protéine THX-NBL1(Cterm), préparée comme décrit à l'exemple 1 ci-dessus, a été évaluée par ELISA indirect vis à vis de sérums issus de porcs infestés par *Trichinella,* en comparaison avec l'antigène d'Excrétion/Sécrétion (E/S) de *T. spiralis.*

L'antigène E/S de référence est préparé selon le protocole décrit par GAMBLE et al., (1988). Il est obtenu à partir du surnageant de culture des larves L1M maintenues en survie pendant 24h dans du RPMI 1640 contenant 1% de pyruvate, 15% de sérum de veau foetal (SVF), 1% de L-glutamine, 100U/ml de pénicilline 100U/ml et 100µg/ml de streptomycine.

Pour les tests ELISA, l'antigène dilué en tampon PBS 1X, à raison de 1,25 µg/ml pour l'antigène E/S et de 2 µg/ml pour THX-NBL1(Cterm), est incubé pendant 1 nuit à 4°C sur des plaques 96 puits (plaques MediSorp, NUNC). Après 3 lavages (PBS 1X, Tween 20 à 0,05%), les plaques sont saturées à température ambiante pendant 1 h avec une solution de lait écrémé dilué à 2 % dans la solution de lavage.

On dépose dans chaque puits 100 µl de sérum de porc dilué au 1/20 en tampon PBS 1X, additionné de Tween 20 à 0,05%. Après incubation pendant 30 min à 37°C, suivie de 3 lavages (PBS 1X, Tween 20 à 0,05%), on dépose dans chaque puits 100 µl de la solution de conjugué (protéine G-peroxidase (P-8170, Sigma) dilué au 1/32000 en tampon PBS 1X, additionné de Tween 20 à 0,05%. Après une nouvelle incubation pendant 30 min à 37°C, suivie de 3 lavages (PBS 1X, Tween 20 à 0,05%), on dépose dans chaque puits 100 µl d'une solution de substrat (3,3',5,5' tetramethylbenzidine-hydrogen peroxide : TMB3). Après 20 min d'incubation à température ambiante à l'obscurité, la réaction est arrêtée par addition de 100 µl/puits de $H_2SO_4$ 0,5 M. La lecture des plaques est effectuée par mesure de l'absorbance à 450 nm.

Le résultat des lectures des plaques ELISA est présenté sous la forme du pourcentage de réactivité d'un sérum échantillon sur un sérum contrôle positif.

$$\% \frac{E}{p} = (OD\ échantillon - OD\ contrôle\ négatif/OD\ contrôle\ positif - OD\ contrôle\ négatif)\ x\ 100$$

Le seuil de positivité (égal à 2 fois la valeur moyenne des échantillons négatifs de référence) est de 14% pour l'ELISA Ag E/S, et 44% pour l'ELISA THX-NBL1 (Cterm).

La cinétique d'apparition des anticorps anti-*Trichinella* détectés par ELISA THX-NBL1 (Cterm) ou par ELISA Ag E/S dans des sérums de porcs conventionnels expérimentalement infestés par 200, 1000 ou 20000 larves L1M de *T. spiralis* a été comparée.

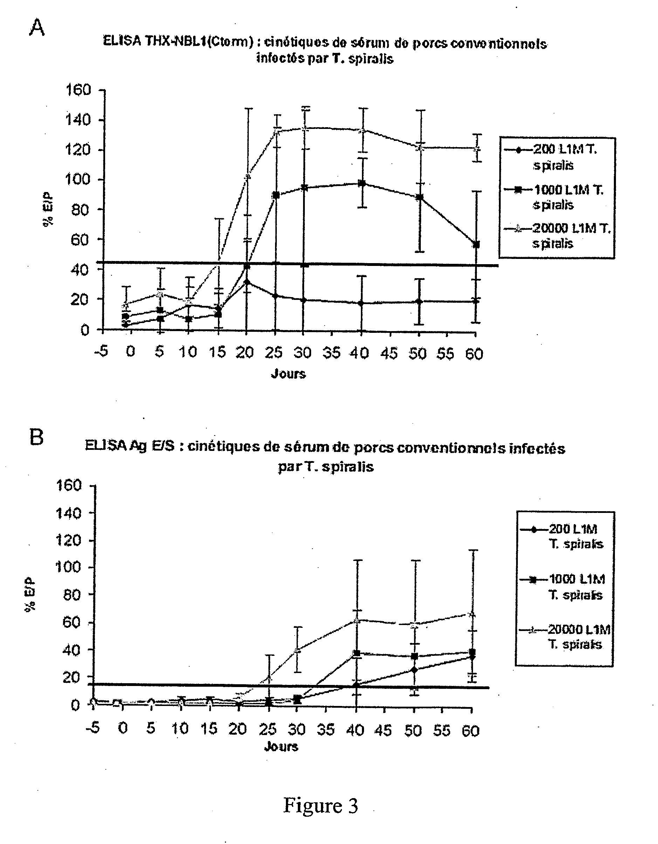

Les résultats sont illustrés par la Figure 3.

L'antigène THX-NBL1(Cterm) permet la détection dose-dépendante des réponses humorales dirigées contre T. *spiralis.* La détection d'épitopes conformationnels par ce test ELISA associée à la détection d'épitopes linéaires par transfert de Western démontrent par ailleurs le caractère immunodominant de la protéine NBL1 de *Trichinella.*

L'ELISA THX-NBL1(Cterm) détecte la séroconversion dès le 25$^e$ jour pi, alors que l'ELISA Ag E/S ne détecte la séroconversion que 10 jours plus tard.

La détection par ELISA Ag E/S, et ELISA THX-NBL1(Cterm) des réponses humorales induites par *T. spiralis* et les trois autres espèces de *Trichinella* identifiées en Europe, *T. nativa, T. britovi* et *T. pseudospiralis,* a également été comparée.

Les résultats sont résumés dans le Tableau I ci-dessous.

TABLEAU I

| Espèce Trichinella | Inoculum (a) | Infectivité (b.) | Taux de dépistage par l'ELISA Ag E/S | Taux de dépistage total par l'ELISA Ag E/S | Taux de dépistage par l'ELISA THX-NBL1 (Cterm) | Taux de dépistage total par l'ELISA THX-NBL1 (Cterm) | Nombre d'animaux co-détectés plus tardivement par l'ELISA THX-NBL1 (Cterm) | Nombre d'animaux détectés au même moment par les 2 tests ELISA | Nombre d'animaux détectés plus précocement par l'ELISA THX-NBL1 (Cterm) | Nombre d'animaux détectés au même moment par les 2 tests ELISA ou plus précocement par l'ELISA THX-NBL1 (Cterm) | Gain de précocité induit par l'ELISA THX-NBL1 (Cterm) (c) |
|---|---|---|---|---|---|---|---|---|---|---|---|
| *T. spiralis* | 200 | 3 | 3/3 | | 1/3 | | | | | | |
| | 1000 | 43,1 | 3/3 | 9/9 | 3/3 | 7/9 | - | - | 7/7 | 7/7 | 5 - 20 |
| | 20000 | 538,9 | 3/3 | | 3/3 | | | | | | |
| *T. britovi* | 200 | 1,8 | 1/3 | | 1/3 | | | | | | |
| | 1000 | 1 | 3/3 | 7/9 | 2/3 | 6/9 | - | - | 6/6 | 6/6 | 5 - 45 |
| | 20000 | 123,1 | 3/3 | | 3/3 | | | | | | |
| *T. nativa* | 200 | 0,0007 | 1/3 | | 1/3 | | | | | | |
| | 1000 | 0,0015 | 1/3 | 5/9 | 2/3 | 6/9 | - | - | 6/6 | 6/6 | 15 - 30 |
| | 20000 | 0,1022 | 3/3 | | 3/3 | | | | | | |
| T. *pseudospiratis* | 200 | 0,058 | 0/3 | | 0/3 | | | | | | |
| | 1000 | 2,1 | 3/3 | 6/9 | 2/3 | 5/9 | - | -- | 5/5 | 5/5 | 5 - 45 |
| | 20000 | 98,9 | 3/3 | | 3/3 | | | | | | |

(a) inoculum infectieux en nombre de L1M de *Trichinella* par porc
(b) charge parasitaire musculaire moyenne par animal (en larves par gramme de muscle)
(c) gain de précocité exprimé en jours obtenu par le test ELISA NBL1(Cterm) comparé au test ELISA Ag E/S chez les animaux co-détectés par ces 2 tests.

L'ensemble de ces résultats montre que l'ELISA THX-NBL1(Cterm) permet une détection particulièrement précoce des réponses humorales, dès le 15e j pi, pour les animaux fortement infestés et une détection légèrement retardée pour les animaux infestés par une charge moyenne de 1000 L1M (25e j pi). Des résultats similaires ont été obtenus avec des porcs holoxéniques infestés selon le même protocole.- La séroconversion la plus précoce détectée par l'ELISA Ag E/S était au 25e j pi. La comparaison des résultats obtenus avec les 2 tests ELISA a démontré un gain de 5 à 20 jours en terme de précocité pour le diagnostic de *T. spiralis* par utilisation de l' ELISA THX-NBL1 (Cterm) (Tableau I). De plus, les animaux infestés par *T. spiralis* qui ont été diagnostiqués à la fois par l'ELISA Ag E/S et l'ELISA THX-NBL1(Cterm) ont vu, pour tous, leur fenêtre de détection sérologique se réduire avec l'ELISA THX-NBL1(Cterm). La sensibilité de l'ELISA THX-NBL1(Cterm) a été démontrée avec le dépistage effectif de 7/9 porcs conventionnels au cours de cette expérimentation animale, soit 3/3 porcs infestés par 20000 L1M, 3/3 porcs infestés par 1000 L1M et 1/3 porc infesté par seulement 200 L1M de *T. spiralis.* Les détections étaient associées à la charge parasitaire musculaire de *T. spiralis* chez ces animaux qui variait en moyenne de 3 larves par gramme (LpG) pour les porcs expérimentalement infestés par 200 L1M, 43 LpG pour les porcs expérimentalement infestés par 1000 L1M, et 538 LpG pour les porcs expérimentalement infestés par 20000 L1M.

Les réponses humorales induites par les trois autres espèces de *Trichinella* identifiées en Europe, *T. nativa, T. britovi* et *T. pseudospiralis,* ont elles aussi été détectées de façon dose-dépendante par l'ELISA THX-NBL1(Cterm), démontrant la conservation génétique et antigénique de NBL1 dans le genre *Trichinella* (immunodominance), et par conséquent tout son intérêt pour le diagnostic à large spectre des trichinelloses. La sensibilité et la précocité du diagnostic (15e j pi) ont été confirmées. La fenêtre de séroconversion a été réduite de 5 à 45 jours avec l'ELISA THX-NBL1(Cterm), et à l'instar de l'infestation par *T. spiralis,* l'ensemble des animaux qui ont été diagnostiqués à la fois par ELISA Ag E/S et ELISA THX-NBL1(Cterm) ont vu leur fenêtre de détection sérologique se réduire avec l'ELISA THX-NBL1(Cterm). De plus, l'analyse de l'infestation par l'espèce *T. nativa* démontre la grande sensibilité de l'ELISA THX-NBL1(Cterm) qui a diagnostiqué 6/9 animaux (contre 5/9 animaux par l'ELISA Ag E/S) dont 2 porcs non dépistés par l'ELISA Ag E/S alors que la charge parasitaire musculaire n'était en moyenne que de $7 \times 10^{-4}$ à 0,1 LpG seulement.

La spécificité de l'ELISA THX-NBL1(Cterm) a été démontrée à l'aide des sérums prélevés sur l'ensemble des animaux avant chaque infestation expérimentale et jusqu'à 10 jours pi. Plus de 200 sérums de porcs plein air ont été utilisés pour montrer la spécificité de la molécule. Aucun porc négatif vis à vis de *Trichinella* ne réagit avec l'ELISA THX-NBL1 (Cterm).

Ces résultats sont illustrés par la Figure 4.

## EXEMPLE 3 : IDENTIFICATION D'UN EPITOPE IMMUNODOMINANT DE NBL1.

Une analyse *in silico* de la séquence en acides aminés déduite de la partie C terminale de NBL1 a été réalisée afin de prédire les régions les plus antigéniques.

Les résultats de l'analyse in *silico* ont conduit à la sélection de 11 peptides chevauchants couvrant 113 AA de la partie C terminale de NBL1 (de l'AA 327 à l'AA 440). Les séquences de ces peptides sont indiquées ci-après

```
N5EM1 :   NH2- NSPEGTVKWASKEDS -CONH2 (SEQ ID NO: 14)
N5EM2 :   NH2- ASKEDSPVDLSTASR -CONH2 (SEQ ID NO: 15)
N5EM3 :   NH2- LSTASRPTNPYTGSR -CONH2 (SEQ ID NO: 16)
N5EM4 :   NH2- PYTGSRPTSPSSGSR -CONH2 (SEQ ID NO: 17)
N5EM5 :   NH2- PSSGSRPTYPSSGSR -CONH2 (SEQ ID NO: 6)
N5EM6 :   NH2- PSSGSRPTSPSSGSR -CONH2 (SEQ ID NO: 18)
N5EM7 :   NH2- PSSGSRPTYPYTGSR -CONH2 (SEQ ID NO: 7)
N5EM8 :   NH2- PYTGSRPTPQKPVFP -CONH2 (SEQ ID NO: 19)
N5EM9 :   NH2- QKPVFPSYQKYPPAV -CONH2 (SEQ ID NO: 20)
N5EM10:   NH2- KYPPAVQKYIDSLPS -CONH2 (SEQ ID NO: 21)
N5EM11:   NH2- RPTSPSSGSRPTYPS -CONH2 (SEQ ID NO: 8)
```

L'antigénicité des peptides N5EM vis-à-vis d'un sérum de porc infesté par 20000 larves L1M de *T. spiralis* et recueilli 60 jours après l'infestation été évaluée par ELISA indirect, en utilisant un protocole identique à celui décrit dans l'Exemple 2, à ceci près que les peptides, préalablement biotinylés sont incubés (2 μg/ml en PBS 1X ; 100 μl/puits) sur des plaques prétraitées à la streptavidine.

Trois peptides immunoréactifs (N5EM5, 7 et 11) ont été détectés. L'analyse de la séquence primaire des peptides immunoréactifs a révélé la présence d'un motif commun de 10 acides aminés (PSSGSRPTYP) (SEQ ID NO: 5). Ce motif est par ailleurs présent 4 fois sur toute la séquence de la protéine NBL1. D'autre part, la cartographie d'épitopes via des peptides chevauchants de 6AA a permis de mettre en évidence l'importance primordiale d'un seul AA, une tyrosine, pour l'immunoréactivité de l'épitope linéaire au sein des peptides.

Des expériences complémentaires ont finalement démontré que N5EM11 était le peptide le plus réactif comparé à N5EM5 et N5EM7 en termes de sensibilité et de précocité.

Ce peptide a été comparé avec l'antigène E/S de *T. spiralis,* comme décrit dans l'Exemple 2 pour THX-NBL1 (Cterm).

Les résultats sont résumés dans le Tableau II ci-dessous :

TABLEAU II

| Espèce *Trichinella* | Inoculum (a). | Infectivité (b). | Taux de dépistage par l'ELISA Ag E/S | Taux de dépistage total par l'ELISA Ag E/S | Taux de dépistage par l'ELISA N5EM11 | Taux de dépistage total par l'ELISA N5EM11 | Nombre d'animaux co-détectés plus tardivement par l'ELISA N5EM11 | Nombre d'animaux détectés au même moment par les 2 tests ELISA | Nombre d'animaux détectés plus précocement par l'ELISA N5EM11 | Nombre d'animaux détectés au même moment par les 2 tests ELISA ou plus précocement par l'ELISA N5EM11 | Gain de précocité induit par l'ELISA N5EM11 (c) |
|---|---|---|---|---|---|---|---|---|---|---|---|
| *T. spiralis* | 200 | 3 | 3/3 | 9/9 | 0/3 | 5/9 | - | 2/5 | 3/5 | 5/5 | 5 -10 |
| | 1000 | 43,1 | 3/3 | | 2/3 | | | | | | |
| | 20000 | 538,9 | 3/3 | | 3/3 | | | | | | |
| *T. britovi* | 200 | 1,8 | 1/3 | 7/9 | 1/3 | 5/9 | - | 1/5 | 4/5 | 5/5 | 5 -30 |
| | 1000 | 1 | 3/3 | | 1/3 | | | | | | |
| | 20000 | 123,1 | 3/3 | | 3/3 | | | | | | |
| *T. nativa* | 200 | 0,0007 | 1/3 | 5/9 | 0/3 | 4/9 | - | - | 4/4 | 4/4 | 15 - 25 |
| | 1000 | 0,0015 | 1/3 | | 1/3 | | | | | | |
| | 20000 | 0,1022 | 3/3 | | 3/3 | | | | | | |
| *T. pseudospiralis* | 200 | 0,058 | 0/3 | 6/9 | 0/3 | 4/9 | 2/4 | - | 2/4 | 2/4 | 30 |
| | 1000 | 2,1 | 3/3 | | 2/3 | | | | | | |
| | 20000 | 98,9 | 3/3 | | 2/3 | | | | | | |

(a) inoculum infectieux en nombre de L1M de *Trichinella* par porc.
(b) charge parasitaire musculaire moyenne par animal (en larves par gramme de muscle).
(c) gain de précocité exprimé en jours obtenu par le test ELISA N5EM11 comparé au test ELISA Ag E/S chez les animaux co-détectés par ces 2 tests.

Ces résultats montrent que l'ELISA N5EM11 permet la détection des infestations modérées à *T. spiralis.* De plus, une détection précoce dès le 20$^{ème}$ jour après infestation a été obtenue, soit 5 à 10 jours plus tôt que ne l'a permis l'ELISA Ag E/S.

On observe également une diminution du taux d'anticorps anti-N5EM11 après le pic de détection, ce qui confirme ce caractère précoce, et qui est utile pour dater une infestation récente. L'ensemble des porcs infestés par *T. spiralis* et diagnostiqués par L'ELISA N5EM11 peptidique présente une fenêtre de séroconversion en partie similaire mais dans la majorité des cas plus réduite que celle observée avec l'ELISA Ag E/S.

Par ailleurs, N5EM11 présente des réactions antigéniques croisées avec le sérum de porcs infestés par *T. nativa, T., britovi* et *T. pseudospiralis* démontrant l'immunodominance de ce peptide du stade L1NN de *Trichinella.* Ces réactions antigéniques croisées conduisent à une détection pouvant être obtenue 5 à 30 jours plus tôt qu'avec l'ELISA Ag E/S.

Une érosion de la sensibilité a néanmoins été notée en comparaison avec l'ELISA Ag E/S, en terme de détection retardée pour des animaux infestés par *T. pseudospiralis* et co-détectés par les deux tests. A *contrario,* l'ELISA N5EM11 a aussi permis de diagnostiquer un animal infesté par *T. nativa* et non dépisté par l'ELISA Ag E/S alors que la charge parasitaire musculaire était résiduelle.

La spécificité de l'ELISA N5EM11 a été évaluée à l'aide de sérums prélevés sur 300 animaux avant infestation expérimentale et jusqu'à 10 jours pi. Cette spécificité est supérieure à 99% (résultats non illustrés).

**EXEMPLE 4 : IDENTIFICATION ET ISOLEMENT DE L'ANTIGENE 411**

Le clone d'ADNc 411 a été sélectionné à partir d'une banque d'ADNc des stades invasifs précoces Ad+L1NN de *T. spiralis.*

La séquence nucléique de ce clone d'ADNc, ainsi que la séquence polypeptidique déduite ont été déterminées, et sont respectivement représentées dans la liste de séquences sous les numéros SEQ ID NO: 3 et SEQ ID NO: 4. Le cadre ouvert de lecture de 411 code pour une protéine putative de 20 kDa. A l'exception d'un peptide signal, aucun domaine protéique n'a été identifié.

La comparaison du cadre ouvert de lecture complet de 411 avec les séquences disponibles sur Genbank montre qu'elle présente 78,7% d'identité avec la protéine Tp21-3 d'Excrétion/Sécrétion identifiée chez *T. pseudospiralis* (AAF79206 ; NAGANO et *al.,* 2001), et 86,6% d'identité avec la séquence de l'ORF 17.20 hypothétique de *T. spiralis* soumise par Polvere et Despommier (AAB48489). Ces comparaisons de séquences identifient 411 comme un nouveau membre de cette famille de gènes commune au genre *Trichinella.*

Le cadre ouvert de lecture complet de 411 a été amplifié à l'aide des oligonucléotides 411F (5'-CACCCGAGAAAACATGCAT-3' )(SEQ ID NO: 22) et 411R (5'-TCCATTCAATTTTGCGTCAC-3') (SEQ ID NO: 23), et l'ADN polymérase AccuPrime *Pfx* DNA polymerase (Invitrogen), et cloné dans le plasmide pET102D/topo, en utilisant le kit d'expression « Champion pET102 Directional TOPO » selon les recommandations du fabricant (Invitrogen).

Le plasmide recombinant obtenu, dénommé pET102-411 code pour une protéine de fusion thioredoxine-411 (THX-411) de 330 AA, pour une masse moléculaire prédite de 36,7 kDa, et portant en position C-terminale une étiquette poly-histidine. La séquence de cette protéine de fusion est représentée sur la Figure 5.

La protéine de fusion THX-411 a été exprimée dans des bactéries *E. coli* BL21 Star (DE3), BL21 (DE3)pLys (Invitrogen) transformées par le plasmide pET102-411, et purifiée par chromatographie d'affinité en conditions dénaturantes sur colonne Ni-NTA (Ni-NTA spin columns kit ; Ni-NTA beads), en utilisant le protocole préconisé par le fournisseur (Qiagen).

La protéine de fusion THX-411 purifiée apparaît, après électrophorèse en conditions dénaturantes (SDS-PAGE) sous forme d'une bande à la taille attendue de 36,7 kDa.

L'immunoréactivité de la protéine THX-411 vis-à-vis d'un sérum de porc indemne de trichinellose, et du sérum du même porc, 30 jours et 50 jours après infestation par 20000 larves L1M de *T. spiralis* a été comparée avec celle de l'antigène de référence E/S (préparé comme décrit à l'exemple 2 ci-dessus). L'analyse a été effectuée par transfert de Western, en utilisant le même protocole que celui décrit à l'exemple 1 ci-dessus.

Les résultats sont illustrés par la Figure 6.

On observe une très forte immunoréactivité de THX-411 avec les sérums du porc infesté par *T. spiralis* et la détection précoce d'anticorps anti-411 (30 jours pi). On observe un léger bruit de fond dû aux réactions croisées entre les protéines bactériennes résiduelles de haute masse moléculaire subsistant dans la préparation de THX-411 et les anticorps anti *E. coli* présents dans les sérums.

**EXEMPLE 5 : UTILISATION DE THX-411 POUR DETECTER LA REPONSE HUMORALE DIRIGEE CONTRE *TRICHINELLA.***

La protéine, préparée comme décrit à l'exemple 4 ci-dessus, a été évaluée par ELISA indirect vis à vis de sérums issus de porcs infestés par *Trichinella,* en comparaison avec l'antigène d'Excrétion/Sécrétion (E/S) de *T. spiralis.*

La cinétique d'apparition des anticorps anti-*Trichinella* détectés par ELISA THX-411 ou par ELISA Ag E/S dans des sérums de porcs conventionnels expérimentalement infestés par 200, 1000 ou 20000 larves L1M de *T. spiralis* a été comparée. La détection par ELISA Ag E/S, et ELISA THX-411 des réponses humorales induites par *T. spiralis* et les trois autres espèces de *Trichinella* identifiées en Europe, *T. nativa, T. britovi* et *T. pseudospiralis,* a également été comparée.

Le protocole utilisé est identique à celui décrit dans l'Exemple 2. L'antigène THX-411 a été utilisé à 2 $\mu$g/ml, et l'antigène E/S à 1,25 $\mu$g/ml.

Le seuil de positivité est de 14% pour l'ELISA Ag E/S, et 52% pour l'ELISA THX-411.

Les résultats sont illustrés par la Figure 7, ainsi que par le Tableau III ci-dessous.

Tableau III

| Espèce *Trichinella* | Inoculum (a). | Infectivité (b). | Taux de dépistage par l'ELISA AgE/S | Taux de dépistage total par l'ELISA AgE/S | Taux de dépistage par l'ELISA THX-411 | Taux de dépistage total par l'ELISA THX-411 | Nombre d'animaux co-détectés plus tardivement par l'ELISA THX-411 | Nombre d'animaux détectés au même moment par les 2 tests ELISA | Nombre d'animaux détectés plus précocement par l'ELISA THX-411 | Nombre d'animaux détectés au même moment par les 2 tests ELISA ou plus précocement par l'ELISA THX-411 | Gain de précocité induit par l'ELISA THX-411 (c) |
|---|---|---|---|---|---|---|---|---|---|---|---|
| *T. spiralis* | 200 | 3 | 3/3 | 9/9 | 1/3 | 5/9 | 2/5 | 1/5 | 2/5 | 3/5 | 5 -10 |
| | 1000 | 43,1 | 3/3 | | 1/3 | | | | | | |
| | 20000 | 538,9 | 3/3 | | 3/3 | | | | | | |
| *T. britovi* | 200 | 1,8 | 1/3 | 7/9 | 0/3 | 4/9 | - | - | 4/4 | 4/4 | 5 -20 |
| | 1000 | 1 | 3/3 | | 2/3 | | | | | | |
| | 20000 | 123,1 | 3/3 | | 2/3 | | | | | | |
| *T. nativa* | 200 | 0,0007 | 1/3 | 5/9 | 1/3 | 6/9 | - | 1/6 | 5/6 | 6/6 | 10 - 20 |
| | 1000 | 0,0015 | 1/3 | | 2/3 | | | | | | |
| | 20000 | 0,1022 | 3/3 | | 3/3 | | | | | | |
| *T. pseudospiralis* | 200 | 0,058 | 0/3 | 6/9 | 0/3 | 2/9 | 1/2 | - | 1/2 | 1/2 | 20 |
| | 1000 | 2,1 | 3/3 | | 0/3 | | | | | | |
| | 20000 | 98,9 | 3/3 | | 2/3 | | | | | | |

(a) inoculum infectieux en nombre de L1M de *Trichinella* par porc.
(b) charge parasitaire musculaire moyenne par animal (en larve par gramme de muscle).
(c) gain de précocité (exprimé en jours) obtenu par le test ELISA 411 comparé au test ELISA Ag E/S chez les animaux co-détectés par ces 2 tests.

EP 1 987 357 B1

L'ensemble de ces résultats montre que l'antigène 411 permet la détection dose-dépendante des réponses humorales dirigées contre *T. spiralis.* La détection d'épitopes conformationnels par ce test ELISA associée à la détection d'épitopes linéaires par transfert de Western démontrent par ailleurs le caractère immunodominant de la protéine 411 de *Trichinella.*

La protéine recombinante THX-411 permet une détection particulièrement précoce des anticorps dirigés contre *T. spiralis* (dès le 20$^e$j pi pour les animaux fortement infestés à 20000 L1M). La séroconversion s'accompagne d'un profil de réponses humorales ayant des titres élevés et maintenus jusqu'à 60j pi. La séroconversion des animaux infestés par une charge modérée et faible du parasite a été détectée plus tardivement au 30$^e$j pi et 60$^e$j pi, respectivement. La séroconversion la plus précoce détectée par l'ELISA Ag E/S est au 25$^e$j pi.

Les animaux infestés par *T. spiralis* qui ont été diagnostiqués à la fois par l'ELISA Ag E/S et l'ELISA THX-411 ont vu, pour 2/5 d'entre eux, leur fenêtre de détection sérologique se réduire avec l'ELISA THX-411, avec un gain de 5 à 10 jours en terme de précocité. La sensibilité de l'ELISA THX-411 a été démontrée avec le dépistage effectif de 5/9 porcs conventionnels au cours de cette expérimentation animale, soit 3/3 porcs infestés par 20000 L1M, 1/3 porcs infestés par 1000 L1M et 1/3 porc infesté par seulement 200 L1M de *T. spiralis.* Les détections étaient associées à la charge parasitaire musculaire de *T. spiralis* chez ces animaux qui variait en moyenne de 3 larves par gramme (LpG) pour les porcs expérimentalement infestés par 200 L1M, 43 LpG pour les porcs infestés par 1000 L1M, et 538 LpG pour les porcs infestés par 20000 L1M.

Les réponses humorales induites par *T. nativa, T. britovi* et *T. pseudospiralis* ont elles aussi été détectées par l'ELISA THX-411 (10/12 animaux infestés par 20000 L1M), démontrant la conservation génétique et antigénique de 411 dans le genre *Trichinella* et par conséquent tout son intérêt pour le diagnostic à large spectre des trichinelloses. La précocité du diagnostic a été confirmée dès le 20$^e$j pi. La fenêtre de séroconversion a été réduite de 5 à 20 jours avec l'ELISA 411, et à l'instar de l'infestation par *T. spiralis,* 50% à 100% des animaux qui ont été diagnostiqués à la fois par ELISA Ag E/S et ELISA THX-411 ont vu leur fenêtre de détection sérologique se réduire avec l'ELISA THX-411. L'analyse de l'infestation par l'espèce *T. nativa* a démontré une sensibilité accrue de l'ELISA THX-411 qui a diagnostiqué 6/9 animaux (contre 5/9 animaux par l'ELISA Ag E/S) alors que la charge parasitaire musculaire n'était en moyenne que de $7x10^{-4}$ à 0,1 LpG seulement. De plus, des profiles de réponses humorales similaires ont été obtenus pour les animaux infestés par *T. spiralis* et *T. nativa,* alors que l'intensité de l'infestation générée par cette dernière espèce était significativement plus faible, suggérant un caractère immunogène très important de la protéine 411 de *T. nativa* et son utilisation pour la détection de cette espèce résistante à la congélation.

La spécificité de l'ELISA THX-411 a été évaluée à l'aide de sérums prélevés sur 150 animaux avant infestation expérimentale et jusqu'à 10 jours pi. Cette spécificité est supérieure à 99% (résultats non illustrés).

## **Conclusion**

Les antigènes NBL1 et 411 constituent des antigènes immunodominants et conservés au sein du genre *Trichinella.* Les tests ELISA utilisant ces antigènes (NBL1 partie C terminale ; épitope peptidique N5EM11 de NBL1 ; antigène 411) ont une spécificité vis-à-vis de *Trichinella* supérieure à 99%, et permettent le diagnostic précoce (15-60 jours après infestation) des trichinelloses porcines produites par les 4 espèces de *Trichinella* identifiées en Europe.

Le test ELISA utilisant la protéine recombinante purifiée THX-NBL1(Cterm), contenant l'épitope immunodominant de NBL1 localisé dans la partie C terminale de la protéine, est spécifique de *Trichinella,* sensible, et permet le diagnostic des trichinelloses porcines avec une précocité accrue de 5 à 45 jours par rapport à L'ELISA Ag E/S. De plus, ce test a permis d'obtenir une fenêtre de détection sérologique réduite par rapport à ELISA Ag E/S chez 100% des animaux qui ont été diagnostiqués à la fois par l'ELISA Ag E/S et par ELISA THX-NBL1 (Cterm).

Le test ELISA utilisant la protéine recombinante purifiée THX-411 reproduit avec une sensibilité actuellement un peu plus réduite (17/36 animaux diagnostiqués) les cinétiques des réponses humorales détectées par l'ELISA Ag E/S. Néanmoins, la sensibilité de ce nouvel ELISA a permis de diagnostiquer un porc non dépisté par l'ELISA Ag E/S. L'ELISA THX-411 peut permettre le diagnostic des trichinelloses porcines avec une précocité accrue de 5 à 20 jours par rapport à l'ELISA Ag E/S. De plus, 50% à 100% des animaux qui ont été diagnostiqués à la fois par ELISA Ag E/S et ELISA THX-411 ont vu leur fenêtre de détection sérologique se réduire avec l'ELISA THX-411.

L'ensemble de ces résultats montre que les antigènes NBL1 et 411 peuvent être utilisés, en alternative à l'antigène E/S, ou en complément de celui-ci, pour le diagnostic sérologique précoce des trichinelloses. En outre, l'association de ces deux nouveaux antigènes de *Trichinella* permet l'amélioration de la sensibilité de l'ELISA par le diagnostic d'un animal supplémentaire.

L'association de NBL1 (Cterm) avec un peptide immunodominant de type N5EM11, et 411 permet d'améliorer la sensibilité du diagnostic de *Trichinella* (24/36 animaux détectés).

L'effet additif de NBL1 avec 411 se traduit par le gain de détection d'un animal induit par 411, ainsi que par la détection plus précoce d'un animal induite par 411. Le tryptique (NB1L, 411, N5EM11) stabilise dans le temps le test ELISA par la codétection simultanée d'anticorps via 2 ou 3 antigènes, sans gain supplémentaire en terme de nombre d'animaux toutefois.

Par contre le diagnostic précoce <30jpi est obtenu grâce aux deux antigènes NBL1 et 411.

RÉFÉRENCES

MURRELL et al., The systematics of the genus Trichinella with a key to species, Vet Parasitol, 93, 293-307, (2000).

GASSER et al., Nonisotopic single-strand conformation polymorphism analysis of sequence variability in ribosomal DNA expansion segments within the genus Trichinella (Nematoda: Adenophorea), Electrophoresis, 25, 3357-3364, (2004).

BOIREAU et al., Risques parasitaires liés aux aliments d'origine animale, Revue française des laboratoires, 71-89, (2002).

CAPO & DESPOMMIER, Clinical aspects of infection with Trichinella spp, Clin Microbiol Rev, 9, 47-54, (1996).

FOURESTIE et al., Randomized trial of albendazole versus tiabendazole plus flubendazole during an outbreak of human trichinellosis, Parasitol Res, 75, 36-41, (1988).

DUPOUY-CAMET, Trichinellosis: a worldwide zoonosis, Vet Parasitol, 93, 191-200, (2000).

MURRELL & POZIO, Trichinellosis: the zoonosis that won't go quietly, Int J Parasitol, 30, 1339-1349, (2000).

BOIREAU et al., Trichinella in horses: a low frequency infection with high human risk, Vet Parasitol, 93, 309-320, (2000).

GAMBLE et al., Diagnosis of swine trichinosis by enzyme-linked immunosorbent assay (ELISA) using an excretory-secretory antigen, Vet Parasitol, 13, 349-361, (1983).

GAMBLE et al., Evaluation of excretory-secretory antigens for the serodiagnosis of swine trichinellosis, Vet Parasitol, 30, 131-137, (1988).

REASON et al., Novel tyvelose-containing tri- and tetra-antennary N-glycans in the immunodominant antigens of the intracellular parasite Trichinella spiralis, Glycobiology, 4, 593-603, (1994).

NAGANO et al., Molecular cloning and characterization of a 21 kDa protein secreted from Trichinella pseudospiralis, J Helminthol, 75, 273-278, (2001).

**Revendications**

1. Utilisation d'un polypeptide antigénique reconnu par des anticorps anti-*Trichinella* comme réactif pour la détection d'anticorps anti-*Trichinella* dans un prélèvement biologique, **caractérisée en ce que** ledit polypeptide comprend un épitope immunodominant de l'antigène NBL1, lequel épitope est défini par la séquence PSSGSRPTYP (SEQ ID NO: 5).

2. Utilisation selon la revendication 1, **caractérisée en ce que** l'on utilise un mélange comprenant un ou plusieurs polypeptide(s) tel(s) que défini(s) dans la revendication 1, optionnellement en combinaison avec un ou plusieurs polypeptide(s) comprenant les acides aminés 25-175 de la séquence SEQ ID NO: 4, ou comprenant une séquence présentant au moins 70% d'identité avec la séquence des acides aminés 25-175 de la séquence SEQ ID NO: 4.

3. Polypeptide antigénique reconnu par des anticorps anti-*Trichinella,* tel que défini dans la revendication 1, à l'exclusion du polypeptide identifié par le numéro d'accès GenBank AAK16520.1, et du polypeptide identifié par le numéro d'accès GenBank AAR36900.1.

4. Polypeptide antigénique selon la revendication 3, contenant une ou plusieurs des séquences suivantes : la séquence : PSSGSRPTYPSSGSR (SEQ ID NO: 6); la séquence PSSGSRPTYPYTGSR (SEQ ID NO: 7); la séquence RPTSPSSGSRPTYPS (SEQ ID NO: 8).

5. Polypeptide antigénique selon la revendication 4, contenant les acides aminés 363-409 de la séquence SEQ ID NO: 2.

6. Polypeptide antigénique selon la revendication 3, **caractérisé en ce qu'**il est constitué par un polypeptide chimérique comprenant une ou plusieurs copie(s) d'un polypeptide tel que défini dans la revendication 1, optionnellement avec une ou plusieurs copie(s) d'un polypeptide tel que défini dans la revendication 2, éventuellement fusionnés à une ou des séquence(s) hétérologue(s).

7. Polynucléotide codant pour un polypeptide antigénique selon une quelconque des revendications 3 à 6.

8. Vecteur recombinant contenant un ou plusieurs polynucléotide(s) selon la revendication 7.

9. Cellule-hôte transformée par un vecteur recombinant selon la revendication 8.

10. Utilisation d'un polypeptide tel que défini dans la revendication 1 pour l'obtention d'une composition utilisable pour la production d'anticorps.

11. Anticorps reconnaissant spécifiquement un polypeptide tel que défini dans la revendication 1.

12. Anticorps selon la revendication 11, **caractérisé en ce qu'**il reconnaît l'épitope PSSGSRPTYP (SEQ ID NO: 5).

**13.** Procédé de détection de la présence d'anticorps anti-*Trichinella* dans un échantillon biologique, lequel procédé est **caractérisé en ce qu'**il comprend :

- la mise en contact dudit échantillon biologique avec un ou plusieurs polypeptide(s) tel(s) que défini(s) dans la revendication 1, optionnellement en combinaison avec un ou plusieurs polypeptides tel(s) que défini(s) dans la revendication 2, dans des conditions permettant la formation d'un complexe antigène/anticorps avec les anticorps anti-*Trichinella* éventuellement présents dans ledit échantillon ;
- la détection, par tous moyens appropriés, du complexe antigène/anticorps éventuellement formé.

**14.** Nécessaire pour la détection de la présence d'anticorps anti-*Trichinella* dans un échantillon biologique, **caractérisé en ce qu'**il comprend un ou plusieurs polypeptide(s) tel(s) que défini(s) dans la revendication 1, optionnellement en combinaison avec un ou plusieurs polypeptide(s) tel(s) que défini(s) dans la revendication 2, et, le cas échéant, des tampons et réactifs appropriés pour la constitution d'un milieu réactionnel permettant la formation d'un complexe antigène/anticorps, et, optionnellement, des moyens de détection dudit complexe antigène/anticorps.

**15.** Nécessaire selon la revendication 14, **caractérisé en ce que** le (s) dit (s) polypeptide (s) est (sont) immobilisé (s) sur un support solide.

**16.** Composition immunogène, comprenant un ou plusieurs polypeptide(s) tels que définis dans la revendication 1, optionnellement en combinaison avec un ou plusieurs polypeptide(s) tel(s) que défini(s) dans la revendication 2, ou un ou plusieurs polynucléotide(s) codant pour le(s)dit(s) polypeptide(s) tel(s) que définis dans la revendication 1, optionnellement en combinaison avec un ou plusieurs polynucléotide(s) codant pour le(s)dit(s) polypeptide(s) tels que définis dans la revendication 2, associé(s) à un ou plusieurs adjuvants permettant d'améliorer la réponse immunitaire.

**17.** Composition immunogène selon la revendication 16, **caractérisé en ce qu'**il s'agit d'un vaccin.

**Claims**

**1.** Use of an antigenic polypeptide recognised by anti-*Trichinella* antibodies as a reagent for detecting anti-*Trichinella* antibodies in a biological sample, **characterised in that** the said polypeptide comprises an immunodominant epitope of the NBL1 antigen, this epitope being defined by the sequence PSSGSRPTYP (SEQ ID NO. 5).

**2.** Use according to claim 1, **characterised in that** a mixture is used comprising one or more polypeptide(s) as defined in claim 1, optionally combined with one or more polypeptide(s) comprising amino acids 25-175 of the sequence SEQ ID NO. 4, or comprising a sequence having at least 70% identity with the sequence of amino acids 25-175 of the sequence SEQ ID NO. 4.

**3.** Antigenic polypeptide recognised by anti-*Trichinella* antibodies as defined in claim 1, excluding the polypeptide identified by GenBank accession number AAK16520.1 and the polypeptide identified by GenBank accession number AAR36900.1.

**4.** Antigenic polypeptide according to claim 3, containing one or more of the following sequences: the sequence PSSGSRPTYPSSGSR (SEQ ID NO. 6); the sequence PSSGSRPTYPYTGSR (SEQ ID NO. 7); the sequence RPTSPSSGSRPTYPS (SEQ ID NO. 8).

**5.** Antigenic polypeptide according to claim 4, containing amino acids 363-409 of the sequence SEQ ID NO. 2.

**6.** Antigenic polypeptide according to claim 3, **characterised in that** it is made up of a chimeric polypeptide comprising one or more copy (copies) of a polypeptide as defined in claim 1, optionally with one or more copy (copies) of a polypeptide as defined in claim 2, optionally fused to one or more heterologous sequence(s).

**7.** Polynucleotide coding for an antigenic polypeptide according to any one of claims 3 to 6.

**8.** Recombinant vector containing one or more polynucleotide(s) according to claim 7.

**9.** Host cell transformed by a recombinant vector according to claim 8.

**10.** Use of a polypeptide as defined in claim 1 for obtaining a composition that can be used for producing antibodies.

**11.** Antibody that specifically recognises a polypeptide as defined in claim 1.

**12.** Antibody according to claim 11, **characterised in that** it recognises the epitope PSSGSRPTYP (SEQ ID NO. 5).

**13.** Process for detecting the presence of anti-*Trichinella* antibodies in a biological sample, said process being **characterised in that** it comprises:

> - contacting the said biological sample with one or more polypeptide(s) as defined in claim 1, optionally combined with one or more polypeptide(s) as defined in claim 2, under conditions that allow the formation of an antigen/ antibody complex with the anti-*Trichinella* antibodies that may be present in said sample;
> - detecting, by any suitable means, the antigen/antibody complex that may be formed.

**14.** Kit for detecting the presence of anti-*Trichinella* antibodies in a biological sample, **characterised in that** it comprises one or more polypeptide(s) as defined in claim 1, optionally combined with one or more polypeptide(s) as defined in claim 2, and optionally buffers and reagents that are suitable for forming a reaction medium that allows the formation of an antigen/antibody complex, and optionally means for detecting said antigen/antibody complex.

**15.** Kit according to claim 14, **characterised in that** the said polypeptide(s) is (are) immobilised on a solid support.

**16.** Immunogenic composition comprising one or more polypeptide(s) as defined in claim 1, optionally combined with one or more polypeptide(s) as defined in claim 2, or one or more polynucleotide(s) coding for said polypeptides as defined in claim 1, optionally combined with one or more polynucleotide(s) coding for said polypeptides as defined in claim 2, associated with one or more adjuvants that allow the immune response to be improved.

**17.** Immunogenic composition according to claim 16, **characterised in that** it is a vaccine.

**Patentansprüche**

**1.** Verwendung eines antigenen Polypeptids, das durch Anti-*Trichinella*-Antikörper erkannt wird, als Reagens für die Detektion von Anti-*Trichinella*-Antikörpern in einer biologischen Probe, **dadurch gekennzeichnet, dass** das Polypeptid ein immundominantes Epitop des Antigens NBL1 umfasst, wobei das Epitop durch die Sequenz PSSGSRPTYP (SEQ ID NO: 5) definiert ist.

**2.** Verwendung nach Anspruch 1, **dadurch gekennzeichnet, dass** man ein Gemisch verwendet, das ein oder mehrere Polypeptid(e), wie es (sie) in Anspruch 1 definiert ist (sind), gegebenenfalls in Kombination mit einem oder mehreren Polypeptid(en), umfassend die Aminosäuren 25 bis 175 der Sequenz SEQ ID NO: 4 oder umfassend eine Sequenz, die wenigstens 70% Identität mit der Sequenz der Aminosäuren 25 bis 175 der Sequenz SEQ ID NO: 4 aufweist, umfasst.

**3.** Antigenes Polypeptid, das durch Anti-*Trichinella*-Antikörper erkannt wird, wie es in Anspruch 1 definiert ist, unter Ausschluss des Polypeptids, das durch die Zugangsnummer GenBank AAK16520.1 identifiziert ist, und des Polypeptids, das durch die Zugangsnummer GenBank AAR36900.1 identifiziert ist.

**4.** Antigenes Polypeptid nach Anspruch 3, das eine oder mehrere der folgenden Sequenzen enthält: die Sequenz: PSSGSRPTYPSSGSR (SEQ ID NO: 6); die Sequenz: PSSGSRPTYPYTGSR (SEQ ID NO: 7); die Sequenz: RPTSPSSGSRPTYPS (SEQ ID NO: 8).

**5.** Antigenes Polypeptid nach Anspruch 4, das die Aminosäuren 363 bis 409 der Sequenz SEQ ID NO: 2 enthält.

**6.** Antigenes Polypeptid nach Anspruch 3, **dadurch gekennzeichnet, dass** es durch ein chimäres Polypeptid, umfassend eine oder mehrere Kopie(n) eines Polypeptids, wie es in Anspruch 1 definiert ist, gegebenenfalls mit einer oder mehreren Kopie(n) eines Polypeptids, wie es in Anspruch 2 definiert ist, gegebenenfalls fusioniert an eine oder mehrere heterologe Sequenz(en), gebildet wird.

**7.** Polynucleotid, das für ein antigenes Polypeptid nach einem der Ansprüche 3 bis 6 codiert.

8. Rekombinanter Vektor, der ein oder mehrere Polynucleotid(e) nach Anspruch 7 enthält.

9. Wirtszelle, die durch einen rekombinanten Vektor nach Anspruch 8 transformiert ist.

10. Verwendung eines Polypeptids, wie es in Anspruch 1 definiert ist, für die Herstellung einer Zusammensetzung, die für die Produktion von Antikörpern verwendbar ist.

11. Antikörper, der spezifisch ein Polypeptid, wie es in Anspruch 1 definiert ist, erkennt.

12. Antikörper nach Anspruch 11, **dadurch gekennzeichnet, dass** er das Epitop PSSGSRPTYP (SEQ ID NO: 5) erkennt.

13. Verfahren zur Detektion des Vorliegens von Anti-*Trichinella*-Antikörpern in einer biologischen Probe, wobei das Verfahren **dadurch gekennzeichnet ist, dass** es umfasst:

   - Inkontaktbringen der biologischen Probe mit einem oder mehreren Polypeptid(en), wie in Anspruch 1 definiert, gegebenenfalls in Kombination mit einem oder mehreren Polypeptid(en), wie in Anspruch 2 definiert, unter Bedingungen, die die Bildung eines Antigen/Antikörper-Komplexes mit den Anti-*Trichinella*-Antikörpern, die gegebenenfalls in der Probe vorliegen, erlauben;
   - Detektion des gegebenenfalls gebildeten Antigen/Antikörper-Komplexes durch jedes geeignete Mittel.

14. Kit für die Detektion des Vorliegens von Anti-*Trichinella*-Antikörpern in einer biologischen Probe, **dadurch gekennzeichnet**, das er ein oder mehrere Polypeptid(e), wie in Anspruch 1 definiert, gegebenenfalls in Kombination mit einem oder mehreren Polypeptid(en), wie in Anspruch 2 definiert, und gegebenenfalls Puffer und Reagentien, die für den Aufbau eines Reaktionsmediums geeignet sind, welches die Bildung eines Antigen/Antikörper-Komplexes erlaubt, und gegebenenfalls Mittel zur Detektion des Antigen/Antikörper-Komplexes umfasst.

15. Kit nach Anspruch 14, **dadurch gekennzeichnet, dass** das (die) Polypeptid(e) auf einem festen Träger immobilisiert ist (sind).

16. Immunogene Zusammensetzung, umfassend ein oder mehrere Polypeptid(e), wie in Anspruch 1 definiert, gegebenenfalls in Kombination mit einem oder mehreren Polypeptid(en), wie in Anspruch 2 definiert, oder ein oder mehrere Polynucleotid(e), das/die für das (die) Polypeptid(e), wie in Anspruch 1 definiert, codiert (codieren), gegebenenfalls in Kombination mit einem oder mehreren Polynucleotid(en), das (die) für das (die) Polypeptid(e), wie in Anspruch 2 definiert, codiert (codieren), assoziiert mit einem Adjuvans oder mehreren Adjuvanzien, die eine Verbesserung der Immunantwort erlauben.

17. Immunogene Zusammensetzung nach Anspruch 16, **dadurch gekennzeichnet, dass** es sich um einen Impfstoff handelt.

MGSDKIIHLTDDSFDTDVLKADGAILVDFWAHWCGPCKMIAPILDEIADEYQGKLTVAKLNI
DHNPGTAPKYGIRGIPTLLLFKNGEVAATKVGALSKGQLKEFLDANLAGSGSGDDDDKLGID
PFTENSPEGTVKWASKEDSPVDLSTASRPTNPYTGSRPTSPSSGSRPTYPSSGSRPTSPS
SGSRPTYPSSGSRPTYPSSGSRPTYPYTGSRPTPQKPVFPSYQKYPPAVQKYIDSLPSGTQG
TLEYTVTQNGVTTTTKGELKLEGKPIPNPLLGLDSTRTGHHHHHH

## Figure 1

M   1   2

40 kDa -
30 kDa -    ◄—— THX-NBL1(Cterm)

20 kDa -

## Figure 2

A

**ELISA THX-NBL1(Cterm) : cinétiques de sérum de porcs conventionnels infectés par T. spiralis**

B

**ELISA Ag E/S : cinétiques de sérum de porcs conventionnels infectés par T. spiralis**

Figure 3

Figure 4

MGSDKIIHLTDDSFDTDVLKADGAILVDFWAHWCGPCKMIAPILDEIADEYQGKLTVAKLNI
DHNPGTAPKYGIRGIPTLLLFKNGEVAATKVGALSKGQLKEFLDANLAGSGSGDDDDKLGID
PFTRENMHCQFILSLLLFSLNVVFFEAAKSLDAVDDELKRCTEKQTEICAQTECKAEDAIMT
DLLLEGESDITDHPDFLLYATCMQRCCARLNGAQVAPLKEEEKRRGPSKLPFQSIFEVADQK
TVERCDETMCKSYRKKYENLVALTSSYKKLRSSQELKDYKQCIERCDAKLNGKGELKLEGKP
IPNPLLGLDSTRTGHHHHHH

## Figure 5

## Figure 6

A

ELISA THX-411 : cinétiques de sérums de porcs conventionnels infectés par
T. spiralis

B

ELISA Ag E/S : cinétiques de sérum de porcs conventionnels infectés
par T. spiralis

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5422263 A
- US 5541075 A
- US 5707817 A
- EP 480982 A
- EP 825875 A
- US 5422109 A
- US 6251407 B
- US 6610309 B
- FR 0601058

**Littérature non-brevet citée dans la description**

- **Boireau et al.** MULTIDISCIPLINARITY FOR PARASITES, VECTORS AND PARASITIC DISEASES. MEDIMOND PUBLISHING CO, 2004, vol. 1, 181-188
- **MURRELL et al.** The systematics of the genus Trichinella with a key to species. *Vet Parasitol,* 2000, vol. 93, 293-307
- **GASSER et al.** Nonisotopic single-strand conformation polymorphism analysis of sequence variability in ribosomal DNA expansion segments within the genus Trichinella (Nematoda: Adenophorea). *Electrophoresis,* 2004, vol. 25, 3357-3364
- Risques parasitaires liés aux aliments d'origine animale. **BOIREAU et al.** Revue française des laboratoires. 2002, 71-89
- **CAPO ; DESPOMMIER.** Clinical aspects of infection with Trichinella spp. *Clin Microbiol Rev,* 1996, vol. 9, 47-54
- **FOURESTIE et al.** Randomized trial of albendazole versus tiabendazole plus flubendazole during an outbreak of human trichinellosis. *Parasitol Res,* 1988, vol. 75, 36-41
- **DUPOUY-CAMET.** Trichinellosis: a worldwide zoonosis. *Vet Parasitol,* 2000, vol. 93, 191-200
- **MURRELL ; POZIO.** Trichinellosis: the zoonosis that won't go quietly. *Int J Parasitol,* 2000, vol. 30, 1339-1349
- **BOIREAU et al.** Trichinella in horses: a low frequency infection with high human risk. *Vet Parasitol,* 2000, vol. 93, 309-320
- **GAMBLE et al.** Diagnosis of swine trichinosis by enzyme-linked immunosorbent assay (ELISA) using an excretory-secretory antigen. *Vet Parasitol,* 1983, vol. 13, 349-361
- **GAMBLE et al.** Evaluation of excretory-secretory antigens for the serodiagnosis of swine trichinellosis. *Vet Parasitol,* 1988, vol. 30, 131-137
- **REASON et al.** Novel tyvelose-containing tri- and tetra-antennary N-glycans in the immunodominant antigens of the intracellular parasite Trichinella spiralis. *Glycobiology,* 1994, vol. 4, 593-603
- **NAGANO et al.** Molecular cloning and characterization of a 21 kDa protein secreted from Trichinella pseudospiralis. *J Helminthol,* 2001, vol. 75, 273-278

SEQUENCE

<110> INSTITUT NATIONAL DE LA RECHERCHE AGRONOMIQUE AGENCE FRANCAISE DE SECURITE SANITAIRE DES ALIMENTS JILIN UNIVERSITY
BOIREAU, Pascal
LIU, Mingyuan
FU, Baoquan
LE RHUN, Danielle
BAHUON, Céline
VALLEE, Isabelle
LE GUERHIER, Franck
HERNANDEZ BELLO, Romel

<120> POLYPEPTIDES RECONNUS PAR DES ANTICORPS ANTI-TRICHINELLA, ET LEURS APPLICATIONS.

<130> MJP/mad-F539/130-PCT

<150> FR 06/01058
<151> 2006-02-07

<160> 23

<170> PatentIn version 3.3

<210> 1
<211> 1609
<212> DNA
<213> Trichinella spiralis

<220>
<221> CDS
<222> (90)..(1487)

<400> 1

```
gaaaagtgcc gctttgtttc aaagacaaat agaaatgaaa caaaggatat tccatacgaa      60

cagtagccat taaaaggtgt gctgcatgc atg cat aaa att aca cac aaa agt       113
                                   Met His Lys Ile Thr His Lys Ser
                                   1               5

att gta tca cgt cat aca ttt gct gtt tat ttg tta gtt agt ggt cag      161
Ile Val Ser Arg His Thr Phe Ala Val Tyr Leu Leu Val Ser Gly Gln
    10              15              20

aaa ctg caa tat ata tat ata ttt att tgc aaa atg att aga cgt ctt      209
Lys Leu Gln Tyr Ile Tyr Ile Phe Ile Cys Lys Met Ile Arg Arg Leu
25              30              35              40

ttt caa tat acc tca atg act ttt gct tgg att ctt ctc ttc tta tcc      257
Phe Gln Tyr Thr Ser Met Thr Phe Ala Trp Ile Leu Leu Phe Leu Ser
            45              50              55

gca gct tct cca tca cta ggg gcg ttt gaa tgc ggt gtg cca cat ttt      305
Ala Ala Ser Pro Ser Leu Gly Ala Phe Glu Cys Gly Val Pro His Phe
            60              65              70

aaa ccc tat ata tgg aaa tct ggt cga att gtt ggt gga act gac gta      353
Lys Pro Tyr Ile Trp Lys Ser Gly Arg Ile Val Gly Gly Thr Asp Val
```

EP 1 987 357 B1

|  | 75 | | | 80 | | | 85 | | |

```
cga cca cac tca cat cca tgg cag att caa ttg tta aag tca gaa acg        401
Arg Pro His Ser His Pro Trp Gln Ile Gln Leu Leu Lys Ser Glu Thr
    90                  95                  100

gga ggc tac agc agc ttg tgc ggt ggt agt ctt gtt cat ttc ggt aaa        449
Gly Gly Tyr Ser Ser Leu Cys Gly Gly Ser Leu Val His Phe Gly Lys
105                 110                 115                 120

ccc tca aat ggt act cga ttt gta ctt acc gcc gcg cac tgt ata act        497
Pro Ser Asn Gly Thr Arg Phe Val Leu Thr Ala Ala His Cys Ile Thr
            125                 130                 135

act agc aat atg tat cca aga acg tca aga ttt aca gtt gtg acc ggt        545
Thr Ser Asn Met Tyr Pro Arg Thr Ser Arg Phe Thr Val Val Thr Gly
            140                 145                 150

gcc cac aac atc aaa atg cat gaa aaa gaa aaa aag cgc ata cca att        593
Ala His Asn Ile Lys Met His Glu Lys Glu Lys Lys Arg Ile Pro Ile
            155                 160                 165

act tcc tat tat gtt cag cac tgg aac cct gtg atg aca aca aac gac        641
Thr Ser Tyr Tyr Val Gln His Trp Asn Pro Val Met Thr Thr Asn Asp
            170                 175                 180

att gcg ttg ctt cgc ctg gca gaa act gtt tat tat aat gaa tat acc        689
Ile Ala Leu Leu Arg Leu Ala Glu Thr Val Tyr Tyr Asn Glu Tyr Thr
185                 190                 195                 200

agg cct gtc tgt ttg cca gaa cca aat gaa gaa ttg act cct gga gat        737
Arg Pro Val Cys Leu Pro Glu Pro Asn Glu Glu Leu Thr Pro Gly Asp
            205                 210                 215

att tgc gtt gtc acc gga tgg ggt gat acg act gaa aat gga act act        785
Ile Cys Val Val Thr Gly Trp Gly Asp Thr Thr Glu Asn Gly Thr Thr
            220                 225                 230

tct aat act ttg aag caa gtt ggt gtc aaa att atg aag aaa gga act        833
Ser Asn Thr Leu Lys Gln Val Gly Val Lys Ile Met Lys Lys Gly Thr
            235                 240                 245

tgt gca aat gtg aga agt gaa gtt att act ttt tgc gct gga gct atg        881
Cys Ala Asn Val Arg Ser Glu Val Ile Thr Phe Cys Ala Gly Ala Met
            250                 255                 260

gag ggt ggt aaa gac agt tgt caa ggt gat tct ggt ggc cca ctg ata        929
Glu Gly Gly Lys Asp Ser Cys Gln Gly Asp Ser Gly Gly Pro Leu Ile
265                 270                 275                 280

tgc aag aaa aat ggg aaa agt gtt caa ttc ggt gtc gtt agt tat ggt        977
Cys Lys Lys Asn Gly Lys Ser Val Gln Phe Gly Val Val Ser Tyr Gly
            285                 290                 295

act gga tgc gcc aga aaa ggt tat ccc gga gtg tat gcc aaa gtt cca       1025
Thr Gly Cys Ala Arg Lys Gly Tyr Pro Gly Val Tyr Ala Lys Val Pro
            300                 305                 310

tca tat gtc aca tgg tta aat aaa gct gca aaa gaa ctt gaa aat tct       1073
Ser Tyr Val Thr Trp Leu Asn Lys Ala Ala Lys Glu Leu Glu Asn Ser
            315                 320                 325
```

20

```
cct gaa gga act gta aaa tgg gct tca aaa gaa gat tcg cca gtc gat        1121
Pro Glu Gly Thr Val Lys Trp Ala Ser Lys Glu Asp Ser Pro Val Asp
    330                 335                 340

tta tct act gca tca aga cca act aac cca tat act ggg tca aga ccg        1169
Leu Ser Thr Ala Ser Arg Pro Thr Asn Pro Tyr Thr Gly Ser Arg Pro
345                 350                 355                 360

aca tct cca tct agt gga tca aga ccc aca tat cca tct agt gga tca        1217
Thr Ser Pro Ser Ser Gly Ser Arg Pro Thr Tyr Pro Ser Ser Gly Ser
                365                 370                 375

aga cca aca tct cca tct agt gga tca aga ccc aca tat cca tct agt        1265
Arg Pro Thr Ser Pro Ser Ser Gly Ser Arg Pro Thr Tyr Pro Ser Ser
            380                 385                 390

gga tca aga ccc aca tat cca tct agt gga tca aga cca aca tat cca        1313
Gly Ser Arg Pro Thr Tyr Pro Ser Ser Gly Ser Arg Pro Thr Tyr Pro
            395                 400                 405

tat act gga tca aga cct act cct caa aag cca gta ttt cca tca tac        1361
Tyr Thr Gly Ser Arg Pro Thr Pro Gln Lys Pro Val Phe Pro Ser Tyr
    410                 415                 420

caa aaa tat ccg cca gca gtt caa aaa tac att gat agt tta cca agc        1409
Gln Lys Tyr Pro Pro Ala Val Gln Lys Tyr Ile Asp Ser Leu Pro Ser
425                 430                 435                 440

gga acg caa gga acg ctc gaa tac aca gtc aca cag aat gga gtt act        1457
Gly Thr Gln Gly Thr Leu Glu Tyr Thr Val Thr Gln Asn Gly Val Thr
                445                 450                 455

aca aca aca tat tat cac ttt tct aag taa aaatattatg attaattcac        1507
Thr Thr Thr Tyr Tyr His Phe Ser Lys
                460                 465

tactgctctg aacgtaatta aaaaaggaat atttattaag cattttaata tgacacatta    1567

tatatattaa aacagtcaaa tttgaaaaaa aaaaaaaaaa aa                        1609


<210> 2
<211> 465
<212> PRT
<213> Trichinella spiralis

<400> 2


    Met His Lys Ile Thr His Lys Ser Ile Val Ser Arg His Thr Phe Ala
    1               5                   10                  15

    Val Tyr Leu Leu Val Ser Gly Gln Lys Leu Gln Tyr Ile Tyr Ile Phe
                20                  25                  30

    Ile Cys Lys Met Ile Arg Arg Leu Phe Gln Tyr Thr Ser Met Thr Phe
                35                  40                  45

    Ala Trp Ile Leu Leu Phe Leu Ser Ala Ala Ser Pro Ser Leu Gly Ala
            50                  55                  60
```

```
Phe Glu Cys Gly Val Pro His Phe Lys Pro Tyr Ile Trp Lys Ser Gly
65              70          75              80

Arg Ile Val Gly Gly Thr Asp Val Arg Pro His Ser His Pro Trp Gln
            85          90              95

Ile Gln Leu Leu Lys Ser Glu Thr Gly Gly Tyr Ser Ser Leu Cys Gly
        100         105             110

Gly Ser Leu Val His Phe Gly Lys Pro Ser Asn Gly Thr Arg Phe Val
        115         120             125

Leu Thr Ala Ala His Cys Ile Thr Thr Ser Asn Met Tyr Pro Arg Thr
    130             135         140

Ser Arg Phe Thr Val Val Thr Gly Ala His Asn Ile Lys Met His Glu
145             150             155             160

Lys Glu Lys Lys Arg Ile Pro Ile Thr Ser Tyr Tyr Val Gln His Trp
            165             170             175

Asn Pro Val Met Thr Thr Asn Asp Ile Ala Leu Leu Arg Leu Ala Glu
        180             185             190

Thr Val Tyr Tyr Asn Glu Tyr Thr Arg Pro Val Cys Leu Pro Glu Pro
        195             200             205

Asn Glu Glu Leu Thr Pro Gly Asp Ile Cys Val Val Thr Gly Trp Gly
    210             215             220

Asp Thr Thr Glu Asn Gly Thr Thr Ser Asn Thr Leu Lys Gln Val Gly
225             230             235             240

Val Lys Ile Met Lys Lys Gly Thr Cys Ala Asn Val Arg Ser Glu Val
            245             250             255

Ile Thr Phe Cys Ala Gly Ala Met Glu Gly Gly Lys Asp Ser Cys Gln
        260             265             270

Gly Asp Ser Gly Gly Pro Leu Ile Cys Lys Lys Asn Gly Lys Ser Val
        275             280             285

Gln Phe Gly Val Val Ser Tyr Gly Thr Gly Cys Ala Arg Lys Gly Tyr
    290             295             300

Pro Gly Val Tyr Ala Lys Val Pro Ser Tyr Val Thr Trp Leu Asn Lys
305             310             315             320

Ala Ala Lys Glu Leu Glu Asn Ser Pro Glu Gly Thr Val Lys Trp Ala
            325             330             335

Ser Lys Glu Asp Ser Pro Val Asp Leu Ser Thr Ala Ser Arg Pro Thr
            340             345             350

Asn Pro Tyr Thr Gly Ser Arg Pro Thr Ser Pro Ser Ser Gly Ser Arg
        355             360             365

Pro Thr Tyr Pro Ser Ser Gly Ser Arg Pro Thr Ser Pro Ser Ser Gly
    370             375             380
```

22

```
Ser Arg Pro Thr Tyr Pro Ser Ser Gly Ser Arg Pro Thr Tyr Pro Ser
385                 390             395                 400

Ser Gly Ser Arg Pro Thr Tyr Pro Tyr Thr Gly Ser Arg Pro Thr Pro
                405             410                 415

Gln Lys Pro Val Phe Pro Ser Tyr Gln Lys Tyr Pro Pro Ala Val Gln
            420             425             430

Lys Tyr Ile Asp Ser Leu Pro Ser Gly Thr Gln Gly Thr Leu Glu Tyr
            435             440             445

Thr Val Thr Gln Asn Gly Val Thr Thr Thr Thr Tyr Tyr His Phe Ser
    450             455             460

Lys
465
```

<210> 3
<211> 700
<212> DNA
<213> Trichinella spiralis

<220>
<221> CDS
<222> (1)..(528)

<400> 3

```
cga gaa aac atg cat tgc caa ttc att ctc tct ttg ctc ctt ttc tct    48
Arg Glu Asn Met His Cys Gln Phe Ile Leu Ser Leu Leu Leu Phe Ser
1               5                   10                  15

ttg aac gtc gta ttc ttc gaa gcc gcg aaa tca ctg gat gcc gta gac    96
Leu Asn Val Val Phe Phe Glu Ala Ala Lys Ser Leu Asp Ala Val Asp
                20                  25                  30

gat gaa tta aaa aga tgt act gag aag caa act gaa att tgt gct caa   144
Asp Glu Leu Lys Arg Cys Thr Glu Lys Gln Thr Glu Ile Cys Ala Gln
            35                  40                  45

aca gaa tgc aaa gca gaa gat gca att atg aca gat ctg ctt ctc gag   192
Thr Glu Cys Lys Ala Glu Asp Ala Ile Met Thr Asp Leu Leu Leu Glu
        50                  55                  60

gga gaa agc gac att act gat cat cct gac ttc ctt tta tac gca act   240
Gly Glu Ser Asp Ile Thr Asp His Pro Asp Phe Leu Leu Tyr Ala Thr
65                  70                  75                  80

tgc atg caa cgt tgc tgt gca aga ctg aac ggc gct caa gta gct cca   288
Cys Met Gln Arg Cys Cys Ala Arg Leu Asn Gly Ala Gln Val Ala Pro
                85                  90                  95

ttg aaa gaa gaa gaa aaa cga aga gga cct tca aaa tta ccg ttc caa   336
Leu Lys Glu Glu Glu Lys Arg Arg Gly Pro Ser Lys Leu Pro Phe Gln
                100                 105                 110

agc att ttt gaa gtt gct gat caa aaa aca gtt gaa aga tgt gat gaa   384
Ser Ile Phe Glu Val Ala Asp Gln Lys Thr Val Glu Arg Cys Asp Glu


            115                 120                 125

aca atg tgc aag agt tat aga aag aaa tat gaa aat ttg gta gca ttg   432
Thr Met Cys Lys Ser Tyr Arg Lys Lys Tyr Glu Asn Leu Val Ala Leu
        130                 135                 140

act tca agc tac aaa aag cta cga tca agc caa gaa ttg aaa gac tac   480
Thr Ser Ser Tyr Lys Lys Leu Arg Ser Ser Gln Glu Leu Lys Asp Tyr
145                 150                 155                 160

aaa caa tgc atc gaa aga tgt gac gca aaa ttg aat gga tta cag taa   528
Lys Gln Cys Ile Glu Arg Cys Asp Ala Lys Leu Asn Gly Leu Gln
                165                 170                 175

agccagatat gaagaatgga gatatgcatt acaaagaaaa attttaactg aaataatttt   588

tgttttataa aatctataaa tatcatttct aactgcatta gaattttttt gaagaaaaat   648

aaaataaaaa aaaaaaaaaa aaaaaaaaaa actcgagggg gggcccggtc cc            700
```

<210> 4
<211> 175
<212> PRT
<213> Trichinella spiralis

<400> 4

```
Arg Glu Asn Met His Cys Gln Phe Ile Leu Ser Leu Leu Leu Phe Ser
1               5                  10        .            15

Leu Asn Val Val Phe Phe Glu Ala Ala Lys Ser Leu Asp Ala Val Asp
            20              25              30

Asp Glu Leu Lys Arg Cys Thr Glu Lys Gln Thr Glu Ile Cys Ala Gln
        35              40              45

Thr Glu Cys Lys Ala Glu Asp Ala Ile Met Thr Asp Leu Leu Leu Glu
        50              55              60

Gly Glu Ser Asp Ile Thr Asp His Pro Asp Phe Leu Leu Tyr Ala Thr
65              70              75              80

Cys Met Gln Arg Cys Cys Ala Arg Leu Asn Gly Ala Gln Val Ala Pro
            85              90              95

Leu Lys Glu Glu Glu Lys Arg Arg Gly Pro Ser Lys Leu Pro Phe Gln
        100             105             110

Ser Ile Phe Glu Val Ala Asp Gln Lys Thr Val Glu Arg Cys Asp Glu
        115             120             125

Thr Met Cys Lys Ser Tyr Arg Lys Lys Tyr Glu Asn Leu Val Ala Leu
    130             135             140

Thr Ser Ser Tyr Lys Lys Leu Arg Ser Ser Gln Glu Leu Lys Asp Tyr
145             150             155             160

Lys Gln Cys Ile Glu Arg Cys Asp Ala Lys Leu Asn Gly Leu Gln
            165             170             175
```

<210> 5
<211> 10
<212> PRT
<213> Trichinella spiralis

<400> 5

```
Pro Ser Ser Gly Ser Arg Pro Thr Tyr Pro
1               5               10
```

<210> 6
<211> 15
<212> PRT
<213> Trichinella spiralis

<400> 6

```
Pro Ser Ser Gly Ser Arg Pro Thr Tyr Pro Ser Ser Gly Ser Arg
1               5               10              15
```

<210> 7
<211> 15
<212> PRT
<213> Trichinella spiralis

<400> 7

```
Pro Ser Ser Gly Ser Arg Pro Thr Tyr Pro Tyr Thr Gly Ser Arg
1               5                   10                      15
```

<210> 8
<211> 15
<212> PRT
<213> Trichinella spiralis

<400> 8

```
Arg Pro Thr Ser Pro Ser Ser Gly Ser Arg Pro Thr Tyr Pro Ser
1               5                   10                      15
```

<210> 9
<211> 46
<212> PRT
<213> Trichinella spiralis

<400> 9

```
Pro Ser Ser Gly Ser Arg Pro Thr Tyr Pro Ser Ser Gly Ser Arg Pro
1               5                   10                  15

Thr Ser Pro Ser Ser Gly Ser Arg Pro Thr Tyr Pro Ser Ser Gly Ser
            20                  25                  30

Arg Pro Thr Tyr Pro Ser Ser Gly Ser Arg Pro Thr Tyr Pro
        35                  40                  45
```

<210> 10
<211> 67
<212> PRT
<213> Trichinella spiralis

<400> 10

```
Ser Arg Pro Thr Asn Pro Tyr Thr Gly Ser Arg Pro Thr Ser Pro Ser
1               5                   10              15

Ser Gly Ser Arg Pro Thr Tyr Pro Ser Ser Gly Ser Arg Pro Thr Ser
            20              25              30

Pro Ser Ser Gly Ser Arg Pro Thr Tyr Pro Ser Ser Gly Ser Arg Pro
        35              40              45

Thr Tyr Pro Ser Ser Gly Ser Arg Pro Thr Tyr Pro Tyr Thr Gly Ser
    50              55              60

Arg Pro Thr
65
```

<210> 11
<211> 134
<212> PRT
<213> Trichinella spiralis

<400> 11

```
Glu Asn Ser Pro Glu Gly Thr Val Lys Trp Ala Ser Lys Glu Asp Ser
1               5                   10              15

Pro Val Asp Leu Ser Thr Ala Ser Arg Pro Thr Asn Pro Tyr Thr Gly
            20              25              30

Ser Arg Pro Thr Ser Pro Ser Ser Gly Ser Arg Pro Thr Tyr Pro Ser
        35              40              45

Ser Gly Ser Arg Pro Thr Ser Pro Ser Ser Gly Ser Arg Pro Thr Tyr
    50              55              60

Pro Ser Ser Gly Ser Arg Pro Thr Tyr Pro Ser Ser Gly Ser Arg Pro
65              70              75              80

Thr Tyr Pro Tyr Thr Gly Ser Arg Pro Thr Pro Gln Lys Pro Val Phe
            85              90              95

Pro Ser Tyr Gln Lys Tyr Pro Pro Ala Val Gln Lys Tyr Ile Asp Ser
            100             105             110

Leu Pro Ser Gly Thr Gln Gly Thr Leu Glu Tyr Thr Val Thr Gln Asn
            115             120             125

Gly Val Thr Thr Thr Thr
            130
```

<210> 12

<211> 22
<212> DNA
<213> Artificial

<220>
<223> NBL1CtermF

<400> 12
caccgaaaat tctcctgaag ga          22


<210> 13
<211> 18
<212> DNA
<213> Artificial


<220>
<223> NBL1CtermR


<400> 13
tgttgttgta gtaactcc          18


<210> 14
<211> 15
<212> PRT
<213> Trichinella spiralis


<400> 14


```
Asn Ser Pro Glu Gly Thr Val Lys Trp Ala Ser Lys Glu Asp Ser
1               5                   10                  15
```


<210> 15
<211> 15
<212> PRT
<213> Trichinella spiralis


<400> 15


```
Ala Ser Lys Glu Asp Ser Pro Val Asp Leu Ser Thr Ala Ser Arg
1               5                   10                  15
```


<210> 16
<211> 15
<212> PRT
<213> Trichinella spiralis


<400> 16


```
Leu Ser Thr Ala Ser Arg Pro Thr Asn Pro Tyr Thr Gly Ser Arg
1               5                   10                  15
```


<210> 17
<211> 15
<212> PRT
<213> Trichinella spiralis


<400> 17

```
        Pro Tyr Thr Gly Ser Arg Pro Thr Ser Pro Ser Ser Gly Ser Arg
        1               5               10              15
```

<210> 18
<211> 15
<212> PRT
<213> Trichinella spiralis

<400> 18

```
        Pro Ser Ser Gly Ser Arg Pro Thr Ser Pro Ser Ser Gly Ser Arg
        1               5               10              15
```

<210> 19
<211> 15
<212> PRT
<213> Trichinella spiralis

<400> 19

```
        Pro Tyr Thr Gly Ser Arg Pro Thr Pro Gln Lys Pro Val Phe Pro
        1               5               10              15
```

<210> 20
<211> 15
<212> PRT
<213> Trichinella spiralis

<400> 20

```
        Gln Lys Pro Val Phe Pro Ser Tyr Gln Lys Tyr Pro Pro Ala Val
        1               5               10              15
```

<210> 21
<211> 15
<212> PRT
<213> Trichinella spiralis

<400> 21

```
        Lys Tyr Pro Pro Ala Val Gln Lys Tyr Ile Asp Ser Leu Pro Ser
        1               5               10              15
```

<210> 22
<211> 19
<212> DNA
<213> Artificial

<220>
<223> 411F

<400> 22
cacccgagaa aacatgcat        19

<210> 23
<211> 20
<212> DNA
<213> Artificial

<220>
<223> 411R

<400> 23
tccattcaat tttgcgtcac        20